# FASCICULE DE BREVET EUROPEEN

(11) **EP 3 218 765 B1**
(45) Date de publication et mention de la délivrance du brevet: **09.10.2024**
(21) Numéro de dépôt: 14827248.7
(22) Date de dépôt: 14.11.2014
(51) Int. Cl.: G02C 13/00, A61B 3/113

(54) **DISPOSITIFS ET PROCÉDÉS POUR LA DÉTERMINATION DE LA POSITION D'UN POINT REMARQUABLE D'UN OEIL ET POUR LE SUIVI DE LA DIRECTION DE REGARD D'UN PORTEUR DE LUNETTES**
VORRICHTUNGEN UND VERFAHREN ZUR BESTIMMUNG DER POSITION EINES CHARAKTERISIERUNGSPUNKTES EINES AUGES UND ZUR VERFOLGUNG DER BLICKRICHTUNG EINES TRÄGERS EINER BRILLE
DEVICES AND METHODS FOR DETERMINING THE POSITION OF A CHARACTERIZING POINT OF AN EYE AND FOR TRACKING THE DIRECTION OF THE GAZE OF A WEARER OF SPECTACLES

(43) Date de publication de la demande: 20.09.2017
(73) Titulaire: Essilor International, 94220 Charenton-le-Pont (FR)
(72) Inventeur: FAYOLLE, Romain, 94227 Charenton-le-Pont (FR); CHENE, Sylvain, 94227 Charenton-le-Pont (FR)
(74) Mandataire: Jacobacci Coralis Harle
(86) Numéro de dépôt international: PCT/FR2014/052919
(87) Numéro de publication internationale: WO 2016/075372

(56) Documents cités:
- EP-A2- 0 125 808
- WO-A1-2011/042623
- WO-A1-2013/121128
- WO-A1-2014/046206
- US-A1- 2014 009 739
- "English translation of WO2014046206", 27 March 2014 (2014-03-27), pages 36, XP055201460, Retrieved from the Internet <URL:www.translationfromenglish.com> [retrieved on 20150709]

## Description

### DOMAINE TECHNIQUE AUQUEL SE RAPPORTE L'INVENTION

La présente invention concerne de manière générale le domaine des dispositifs et procédé de détermination de la position d'un point remarquable d'un oeil d'un individu ainsi que les dispositifs et procédés de suivi de la direction de regard d'un individu.

Elle trouve une application particulièrement avantageuse pour la mise en oeuvre d'un procédé de détermination des zones d'usure de lentilles ophtalmiques pour un porteur d'une monture de vision.

### ARRIERE-PLAN TECHNOLOGIQUE

Dans le cadre de la conception de lentilles ophtalmiques de correction visuelles personnalisées, on cherche à prendre en compte des paramètres géométrico-posturaux individuels, dits de conception optique personnalisée, attachés au porteur et à la monture qu'il a choisie.

Les paramètres géométrico-posturaux recherchés sont liés à la fois à la géométrie de la tête du porteur et à celle de la monture de lunettes choisie, ainsi qu'à la posture du porteur et à son comportement visuel.

Afin de déterminer ces paramètres, on détermine notamment les valeurs des grandeurs suivantes : hauteur des yeux du porteur par rapport au bord inférieur de la lentille ou de la monture de lunettes, distance inter-pupillaires, position des centres de rotation des yeux, coefficient oeil-tête - c'est-à-dire rapport entre l'angle de rotation de la tête et l'angle de rotation des yeux lors de la lecture d'un texte, ou plus généralement, pendant un stimulus visuel excentré par rapport à un axe visuel de référence correspondant à une direction de regard droit devant, valeur de l' « inset » pour une lentille ophtalmique progressive, distance entre la face arrière de la lentille ophtalmique et le sommet de la cornée de l'oeil.

L' « inset » est défini dans la norme ISO13666 : 2012 comme la distance horizontale entre la croix de montage et le centre de la zone de conception de vision de près. L' « inset » est aussi appelé « déport interne ». Il dépend du comportement visuel du porteur.

Les lentilles ophtalmiques progressives permettent au porteur de bénéficier d'une compensation de puissance optique adaptée pour différentes distances de vision sans changer de lunettes. Elles peuvent également corriger d'autres défauts visuels, comme par exemple l'astigmatisme.

Une lentille ophtalmique progressive présente une puissance variable sur la surface de la lentille.

Il est par exemple prévu une première zone de vision pour la vision de loin présentant une première valeur de puissance moyenne, une seconde zone de vision pour la vision de près présentant une seconde valeur de puissance moyenne et, entre ces deux zones, une troisième zone de vision pour la vision intermédiaire, dont la courbure varie progressivement et qui est appelée couloir de progression.

La hauteur de montage de la lentille ophtalmique correspond à la hauteur, par rapport au bord inférieur du cercle de la monture, de la projection de la pupille du porteur ayant une direction de regard primaire prédéterminée sur un plan moyen de ce cercle de la monture choisie, correspondant à une surface moyenne ou à un plan moyen de la lentille ophtalmique une fois montée dans ladite monture.

Cette direction de regard primaire prédéterminée correspond à la direction de regard du porteur dans des conditions de vision de loin.

Ainsi, les positions des zones de vision de loin et de près du porteur sont des paramètres très important pour la conception personnalisée des lentilles ophtalmiques progressives. Ces zones de visions de loin et de près du porteur constituent deux zones d'usure de la lentille ophtalmique.

On connaît notamment des dispositifs de suivi de la direction de regard du porteur appartenant à des dispositifs de mesure optique de type colonne de mesure ou tablette de mesure.

Cependant, ces dispositifs de suivi de la direction de regard présentent l'inconvénient de ne pas permettre un suivi de cette direction de regard dans des conditions de posture et de comportement visuel naturelle, c'est-à-dire non contraint, du porteur, puisque celui-ci doit soit se tenir debout ou assis devant la colonne de mesure soit porter la tablette de mesure dans ses mains.

On connait également des documents WO2014/046206 et US2014/009739 des dispositifs pour déterminer la direction de regard d'un individu portant une paire de lunettes. On connait de plus du document WO2011/042623 un clip de lunettes ainsi qu'une caméra située loin du porteur de lunettes.

### OBJET DE L'INVENTION

Afin de remédier à l'inconvénient précité de l'état de la technique, la présente invention propose un dispositif de détermination de la position d'un point remarquable d'un oeil du porteur dans des conditions de posture et de comportement visuel naturel du porteur, qui peut être utilisé dans un dispositif de suivi de la direction de regard du porteur apte à permettre ce suivi dans des conditions de posture et de comportement visuel naturel du porteur.

Plus particulièrement, on propose selon l'invention un dispositif pour la détermination de la position d'un point remarquable d'un oeil d'un porteur équipé d'une monture de vision tel que décrit dans la revendication 1.

Ainsi, le dispositif de détermination de la position d'un point remarquable de l'oeil du porteur selon l'invention est adapté à permettre la détermination de cette position dans des conditions de comportement visuel et de posture naturelles pour ce porteur, c'est-à-dire non contraintes.

En particulier, le dispositif de détermination de la position du point remarquable de l'oeil peut être au moins partiellement monté sur la monture de vision de l'individu concerné.

Cette monture de vision est de préférence soit la paire de lunettes habituelles de l'individu, munie de ses lentilles ophtalmiques de correction. Il peut également s'agir d'une paire de lunettes nouvellement choisie par cet individu, munie ou non de lentilles ophtalmiques correctrices ou non.

La monture de vision peut également comprendre une monture de lunettes cerclée ou non. Elle peut être du type semi-cerclée à fil de nylon ou de type percée, sans cercle. Il peut également s'agir d'un masque ou de lunettes de réalité augmentée. Elle comporte une ou deux branches et peut être munie d'une ou deux lentilles ophtalmiques ou d'un ou deux dispositifs d'affichage.

La détermination de la position du point remarquable de l'oeil permet la détermination de la direction de regard pendant différentes taches de l'individu.

En outre, le dispositif de détermination de la position d'un point remarquable peut être utilisé pour mesurer d'autres grandeurs telles que la hauteur des yeux du porteur par rapport au bord inférieur de la lentille ou de la monture de lunettes, la distance inter-pupillaires, la position des centres de rotation des yeux, la distance entre la face arrière de la lentille ophtalmique et le sommet de la cornée de l'oeil.

Les valeurs de ces grandeurs permettent à leur tour de déterminer les paramètres géométrico-posturaux nécessaires à la conception optique des lentilles ophtalmiques destinées au porteur et à la monture choisie.

D'autres caractéristiques non limitatives et avantageuses du dispositif conforme à l'invention sont décrites dans les revendications 2 à 10.

En outre, avantageusement, il est prévu que :
- lesdits moyens de positionnement sont adaptés à positionner ledit dispositif par rapport à ladite monture de vision de telle manière que, lorsque ladite monture de vision est disposée en position utile sur la tête du porteur, la source lumineuse est adaptée à éclairer l'oeil du porteur ;
- la monture de vision comprenant au moins une branche pour son installation sur la tête du porteur, les moyens de positionnement sont adaptés à coopérer avec la monture de vision du porteur, de telle sorte que chaque source lumineuse et chaque appareil de capture d'image est disposé du côté de la monture de vision où s'étend ladite branche ;
- ladite monture de vision comportant au moins un cercle et/ou une lentille ophtalmique, chaque source lumineuse et chaque appareil de capture d'image est agencé de telle sorte qu'il est disposé en vis-à-vis de ce même cercle ou de cette même lentille ophtalmique de la monture de vision lorsque les moyens de positionnement coopèrent avec ladite monture de vision ;
- ladite monture de vision comportant au moins deux cercles et/ou deux lentilles ophtalmiques, il est prévu au moins deux sources lumineuses et deux appareils de capture d'image, agencés de telle sorte qu'au moins l'une des deux sources lumineuses et l'un des deux appareils de capture d'images est disposé en vis-à-vis de chaque cercle ou de chaque lentille ophtalmique de la monture de vision lorsque les moyens de positionnement coopèrent avec ladite monture de vision ;
- il est prévu quatre appareils de capture d'image agencés de telle sorte que deux appareils de capture d'images sont disposés en vis-à-vis de chaque cercle ou de chaque lentille ophtalmique de la monture de vision lorsque les moyens de positionnement coopèrent avec la monture de vision ;
- lesdits moyens de positionnement sont des moyens de positionnement qui rendent le dispositif amovible en autorisant le montage du dispositif sur la monture de vision et le démontage de ce dispositif ;
- ledit anneau de support est ouvert ou fermé ;
- chaque source lumineuse est une diode électroluminescente émettant dans le domaine des longueurs d'onde infra-rouges.

L'invention concerne également un dispositif pour le suivi de la direction de regard d'un porteur équipé d'une monture de vision tel que décrit dans la revendication 11.

Ainsi, le dispositif de suivi de la direction du regard selon l'invention est adapté à permettre la détermination de cette direction de regard dans des conditions de comportement visuel et de posture naturelles pour l'individu, c'est-à- dire non contraintes.

En particulier, le dispositif de suivi de la direction du regard peut être au moins partiellement monté sur la monture de vision de cet individu. Cette monture de vision est de préférence soit la paire de lunettes habituelles de l'individu, munie de ses lentilles ophtalmiques de correction. Il peut également s'agir d'une paire de lunettes nouvellement choisie par cet individu, munie ou non de lentilles ophtalmiques correctrices ou non.

La monture de vision peut également comprendre une monture de lunettes cerclée ou non. Elle peut être du type semi-cerclée à fil de nylon ou de type percée, sans cercle. Il peut également s'agir d'un masque ou de lunettes de réalité augmentée. Elle peut être aussi une lunette d'essai ISO 12867 :1998. Elle comporte une ou deux branches et peut être munie d'une ou deux lentilles ophtalmiques ou d'un ou deux dispositifs d'affichage.

Le suivi des directions de regard pendant différentes taches de l'individu permet par exemple de déterminer avec précision les zones d'usure des lentilles ophtalmiques destinées à équiper la paire de lunettes de l'individu, notamment les zones de vision de près et de loin de l'individu.

Les zones d'usure correspondent aux zones de la lentille ophtalmique dans lesquels se trouvent les points d'intersection de la direction de regard du porteur et de la lentille ophtalmique lorsque le porteur balaye du regard une partie de son environnement.

En outre, le dispositif de suivi de la direction du regard peut être utilisé pour mesurer d'autres grandeurs telles que la hauteur des yeux du porteur par rapport au bord inférieur de la lentille ou de la monture de lunettes, la distance inter-pupillaires, la position des centres de rotation des yeux, la distance entre la face arrière de la lentille ophtalmique et le sommet de la cornée de l'oeil.

Les valeurs de ces grandeurs permettent à leur tour de déterminer les paramètres géométrico-posturaux nécessaires à la conception optique des lentilles ophtalmiques destinées au porteur et à la monture choisie.

L'invention propose également un procédé de détermination de la position d'un point remarquable d'au moins un oeil d'un porteur équipé d'une monture de vision, tel que décrit dans la revendication 12.

Ainsi, le procédé selon l'invention autorise la détermination de la position du point remarquable de l'oeil du porteur dans des conditions de posture et de comportement visuel habituelles de l'individu.

Il est alors possible d'en déduire les directions de regard du porteur pendant différentes taches de l'individu ce qui permet de déduire avec précision la position et l'étendue des zones de vision de près et de loin de l'individu sur la lentille ophtalmique correspondante destinées au porteur et à la monture choisie par celui-ci.

D'autres caractéristiques non limitatives et avantageuses de ce procédé conforme à l'invention sont décrites dans les revendications 13 et 14.

L'invention concerne également un procédé de détermination de la direction de regard d'au moins un oeil d'un porteur équipé d'une monture de vision, tel que décrit dans la revendication 15.

Ainsi, le procédé selon l'invention autorise la détermination de la direction de regard du porteur dans des conditions de posture et de comportement visuel habituelles de l'individu.

La détermination des directions de regard pendant différentes taches de l'individu permet de déduire avec précision la position et l'étendue des zones de vision de près et de loin de l'individu sur la lentille ophtalmique correspondante destinées au porteur et à la monture choisie par celui-ci.

D'autres caractéristiques non limitatives et avantageuses de ce procédé conforme à l'invention sont les suivantes :
- à l'étape e), on détermine par le calcul la direction de regard, à partir d'un modèle de l'oeil prédéterminé ;
- le point remarquable de l'oeil dont la position est déterminée à l'étape d) étant le centre de la pupille de l'oeil ou un point du contour de la pupille de l'oeil, à l'étape e), on détermine la position d'un centre de rotation de l'oeil ou d'un centre de la courbure de la cornée de l'oeil, et on en déduit la direction de regard comme étant la droite reliant ce centre de rotation de l'oeil ou ce centre de la courbure de la cornée et le centre de la pupille ou le point du contour de la pupille ;
- le dispositif pour le suivi de la direction de regard comporte au moins trois sources lumineuses en vis-à-vis de l'oeil du porteur et,
à l'étape d) :
- on identifie les reflets cornéens des trois sources lumineuses ainsi que l'image du point remarquable de l'oeil sur l'image capturée à l'étape c)
- on détermine la position relative du point remarquable et des reflets cornéens,
à l'étape e) :
- on déduit la direction du regard du porteur dans un référentiel prédéterminé de la position des sources lumineuses dans ce référentiel prédéterminé de la position relative du point remarquable et des reflets cornéens.

Selon un procédé non revendiqué de détermination d'une zone d'usure d'une lentille ophtalmique par un porteur d'une monture de vision comprenant au moins une monture et/ou une lentille ophtalmique, selon lequel
- on détermine une pluralité de directions du regard de ce porteur selon le procédé de détermination de la direction du regard tel que décrit précédemment,
- on détermine, pour chaque direction du regard, son point d'intersection avec une surface prédéterminée relative à la monture et/ou à la lentille ophtalmique du porteur,
- on détermine ladite zone d'usure en fonction desdits points d'intersection.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique de face d'une paire de lunettes équipées d'un premier mode de réalisation du dispositif de suivi du regard selon l'invention,
- la figure 2 est une vue schématique de face d'une paire de lunettes équipées d'un deuxième mode de réalisation du dispositif de suivi du regard selon l'invention,
- la figure 3 est une vue schématique en perspective arrière d'une paire de lunettes équipées d'un troisième mode de réalisation du dispositif de suivi du regard selon l'invention,
- la figure 4 est une vue schématique partielle du dispositif de suivi du regard de la figure 2,
- les figures 5 à 7 illustrent différentes variantes de mise en oeuvre du procédé de suivi des directions de regard selon l'invention.

### Dispositif

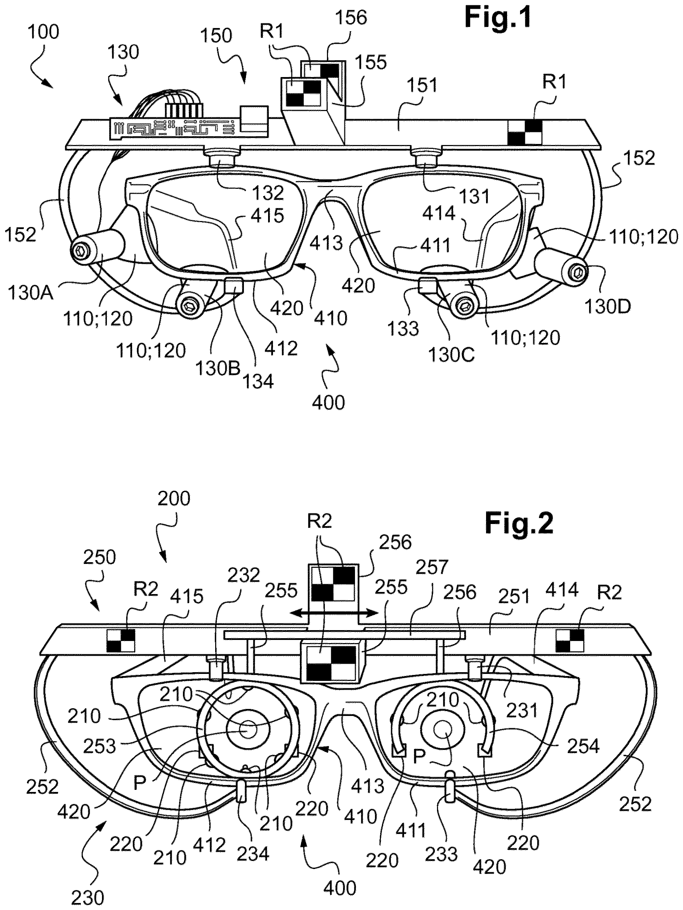

Sur les figures 1 à 3, on a représenté plusieurs modes de réalisation du dispositif de suivi de la direction de regard 100 ; 200 ; 300 selon l'invention.

Comme expliqué ultérieurement, chacun de ces modes de réalisation correspond à un mode de réalisation du dispositif de détermination de la position d'un point remarquable de l'oeil du porteur.

Le porteur est un individu quelconque équipé d'une monture de vision.

Ce dispositif de suivi de la direction de regard 110 ; 200 ; 300 est adapté à être fixé sur la monture de vision du porteur. Il s'agit ici d'une paire de lunettes 400 du porteur.

Cette paire de lunettes 400 comporte ici, comme représenté notamment sur les figures 1 à 3, une monture 410 de lunettes choisie par le porteur et deux lentilles ophtalmiques 420.

Il peut s'agir de la paire de lunette choisie par le porteur lors de l'essayage et ne comportant pas de lentilles ophtalmiques correctrices, de la paire de lunettes habituelle du porteur comportant déjà des lentilles ophtalmiques correctrices adaptée à sa vision, ou d'une paire de lunettes d'essai comportant des lentilles ophtalmiques correctrices adaptée à la vision du porteur.

Dans l'exemple illustré, la monture 410 de lunettes est de type cerclé, c'est-à-dire que la monture 410 comporte des cercles 411, 412 dans lesquels les lentilles ophtalmiques 420 sont montées.

Ces deux cercles 411, 412 sont reliés de manière rigide par un pont nasal 413. Ce pont nasal 413 comporte deux surfaces d'appui sur les ailes du nez du porteur.

Chaque cercle 411, 412 est également relié à une branche 414, 415, habituellement articulée sur le cercle correspondant.

On considérera dans la suite que les branches sont fixes, dans leur position ouverte l'une par rapport à l'autre, c'est-à-dire dans la position adaptée au placement de la paire de lunettes en position utile sur la tête du porteur.

Dans cette position utile, qui est la position de portée de la paire de lunette 400, les branches 414, 415 de la monture 410 reposent chacune sur l'une des oreilles du porteur et le pont nasal 413 de la monture 410 repose sur le nez du porteur.

Chaque cercle 411, 412 de la monture 410 est alors placé en regard de l'un des yeux du porteur, de manière à ce que la lentille ophtalmique correspondante soit elle-même disposée en regard d'un oeil du porteur.

Le porteur regarde à travers ces lentilles ophtalmiques.

La monture 410 représentée sur les exemples des figures est ici une monture en plastique. En conséquence, le pont nasal 413 comporte deux surfaces d'appui sur les ailes du nez du porteur qui sont ici fixes. En variante, la monture peut être une monture métallique. Les surfaces d'appui sur le nez du porteur sont alors portées par deux plaquettes chacune reliée au pont nasal par un bras. La position relative de ces plaquettes est alors réglable.

En variante, la monture de vision peut également comprendre une monture de lunettes non cerclée. Elle peut être du type semi-cerclée à fil de nylon ou de type percée, sans cercle.

Dans ce dernier cas, les lentilles ophtalmiques sont percées de trous de perçage et maintenues chacune par une extrémité du pont nasal et une extrémité de la branche associée à la lentille ophtalmique, qui coopèrent avec les trous de perçage de lentilles. Ce type de monture est similaire à celui décrit précédemment, sauf qu'il ne comporte pas de cercles. Le pont nasal et les branches sont similaires.

La monture de vision peut également être constituée d'un masque ou de lunettes de réalité augmentée ou de lunettes d'essai. Elle comporte une ou deux branches et peut être munie d'une ou deux lentilles ophtalmiques ou d'un ou deux dispositifs d'affichage.

Le dispositif de suivi de la direction de regard 100 ; 200 ; 300 comprend, de manière générale, un dispositif pour la détermination de la position d'un point remarquable d'un oeil du porteur et des moyens de détermination de la direction de regard du porteur en fonction de la position de ce point remarquable de l'oeil.

Plus précisément, le dispositif pour la détermination de la position d'un point remarquable d'un oeil du porteur comprend au moins un appareil de capture d'image 120 ; 220 ; 320.

Il comprend en outre des moyens de positionnement pour positionner l'appareil de capture d'image 120 ; 220 ; 320 par rapport à ladite paire de lunettes, de telle manière que, lorsque ladite paire de lunettes 400 est disposée en position utile sur la tête du porteur, l'appareil de capture d'image 120 ; 220 ; 320 est adaptée à capturer une image de cet oeil du porteur.

Le dispositif de détermination de la position d'un point remarquable de l'oeil du porteur comprend enfin des moyens de détermination de cette position à partir de ladite au moins une image de l'oeil du porteur capturée par l'appareil de capture d'image.

De préférence, comme cela est le cas sur les exemples des figures, le dispositif de détermination de la position d'un point remarquable de l'oeil comprend également au moins une source lumineuse 110 ; 210 ; 310.

Les moyens de positionnement sont alors adaptés pour positionner la source lumineuse 110 ; 210 ; 310 par rapport à ladite paire de lunettes, de telle manière que, lorsque ladite paire de lunettes 400 est disposée en position utile sur la tête du porteur, ladite au moins une source lumineuse 110 ; 210 ; 310 est adaptée à éclairer au moins un oeil du porteur.

Le dispositif de détermination de la position d'un point remarquable de l'oeil du porteur est distinct de la monture de vision du porteur. Il s'agit d'un dispositif amovible, en ce sens que les moyens de positionnement de ce dispositif sur la monture de vision autorisent le montage du dispositif sur la monture de vision, de manière à fixer le dispositif sur la monture de vision, et le démontage de ce dispositif, de manière à dissocier le dispositif de la monture de vision.

L'appareil de capture d'image 120 ; 220 ; 320 est alors adapté à capturer une image de cet oeil du porteur éclairé par ladite source lumineuse 110 ; 210 ; 310.

Les moyens de détermination de la position du point remarquable de l'oeil sont alors programmés pour déterminer cette position à partir de ladite au moins une image de l'oeil du porteur capturée par l'appareil de capture d'image lorsque cet oeil est éclairé par la source lumineuse 110 ; 210 ; 310.

Les moyens de détermination de la direction de regard du porteur sont adaptés à déterminer la direction de regard du porteur en fonction de la position du point remarquable, donc à partir de ladite au moins une image de l'oeil du porteur capturée par l'appareil de capture d'image lorsque cet oeil est éclairé par la source lumineuse 110 ; 210 ; 310.

Plus précisément, dans les exemples représentés sur les figures 1 à 4 et décrits ici, les moyens de positionnement sont adaptés à coopérer avec la paire de lunettes 400 du porteur pour positionner chaque source lumineuse 110 ; 210 ; 310 et chaque appareil de capture d'image 120 ; 220 ; 320.

En pratique, ces moyens de positionnement comporte ici un support de mesure 150 ; 250 ; 350 muni (figures 1 à 3) d'une barre principale 151 ; 251 ; 351 adaptée à être disposée au dessus de la paire de lunettes 400 et de deux bras latéraux 152 ; 252 ; 352, et des moyens de montage 130 ; 230 ; 330 de ce support de mesure 150 ; 250 ; 350 sur la paire de lunettes.

La barre principale 151 ; 251 ; 351 se présente sous la forme d'une tige droite et rigide. Elle est destinée à s'étendre sensiblement dans un plan moyen des lentilles ophtalmiques 420 montées dans la monture 410 qui correspond au plan moyen des cercles 411, 412 de la monture 410 dans le cas de la monture de type cerclée représentée sur les figures.

En variante, cette barre principale peut se présenter sous la forme d'une tige courbée d'une tige comportant deux parties articulées destinées à être disposées en correspondance avec chaque cercle de la monture ou avec chaque lentilles ophtalmiques de la paire de lunettes.

Cette barre principale 151 ; 251 ; 351 présente une longueur légèrement supérieure à la largeur habituelle hors tout des montures de lunettes. Cette largeur hors tout correspond généralement à la largeur mesurée entre les faces externes des branches 414, 415 de la monture 410.

Les bras latéraux 152 ; 252; 352 s'étendent chacun à partir d'une extrémité libre de la barre principale 151 ; 251 ; 351, d'un même côté de cette barre principale 151 ; 251 ; 351.

De cette manière, les bras latéraux 152 ; 252 ; 352 sont adaptés à entourer partiellement les cercles 411, 412 de la monture 410 ou les lentilles ophtalmiques 420.

Chaque bras latéral 152 ; 252 ; 352 se présente sous la forme d'une lame ressort fixée à l'une de ses extrémités sur la barre principale 151 ; 251 ; 351. Les moyens de montage 130 ; 230 ; 330 du support de mesure 150 ; 250 ; 350 sont disposés pour une partie sur la barre principale 151 ; 251 ; 351 et pour une autre partie à l'extrémité libre de chaque bras latéral 132 ; 232 ; 332.

Plus précisément, ces moyens de montage 130 ; 230 ; 330 comprennent d'une part deux clips 131, 132 ; 231, 232 ; 331, 332 supérieurs s'étendant à partir de la barre principale 151 ; 251 ; 351 et, d'autre part, deux clips 133, 134 ; 233, 234 ; 433, 434 inférieurs s'étendant chacun à l'extrémité libre de l'un des bras latéraux 152 ; 252 ; 352.

Chaque clip 131, 132, 133, 134 ; 231, 232, 233, 234 ; 331, 332, 433, 434 comprend ici deux petits cônes en plastique souple constituant une pince adaptée à venir s'accrocher sur les cercles 411, 412 de la monture 410 ou sur les lentilles ophtalmiques si la monture est du type percé. En pratique une partie de la monture ou de la lentille est insérée entre les deux petits cônes qui se déforment pour permettre cette insertion. Ils exercent alors une pression suffisante sur la monture ou les lentilles ophtalmiques pour maintenir en place le support de mesure 150 ; 250 ; 350 sur la paire de lunettes.

Quel que soit le mode de réalisation envisagé ici, les moyens de montage 130 ; 230 ; 330 du support de mesure 150 ; 250 ; 350 sont adaptés au montage dudit support de mesure sur la paire de lunettes 400 du porteur, de telle sorte que chaque source lumineuse 110 ; 210 ; 310 et chaque appareil de capture d'image 120 ; 220 ; 320 du support de mesure 150 ; 250 ; 350 est disposé entre les branches 414 ; 415 de la paire de lunettes 400.

En d'autres termes, les moyens de montage 130 ; 230 ; 330, les sources lumineuses 110 ; 210 ; 310 et les appareils de capture d'image 120 ; 220 ; 320 sont agencées de manière à s'étendre entre les yeux du porteur et les cercles 411, 412 de la monture 410, ou les lentilles ophtalmiques lorsque la monture est du type percé, lorsque le support de mesure 150 ; 250 ; 350 est monté sur la paire de lunettes 400 et la paire de lunettes 400 placée sur le visage du porteur.

Ainsi, de manière générale, dans le cas où la monture de vision ne comprendrait qu'une seule branche pour son installation sur la tête du porteur, les moyens de positionnement serait ici adaptés à coopérer avec cette monture de vision, de telle sorte que chaque source lumineuse et chaque appareil de capture d'image soit disposée du côté de la monture de vision où s'étend ladite branche.

En variante, on peut envisager que les moyens de montage du support de mesure sont adaptés au montage dudit support de mesure sur la monture de vision du porteur, de telle sorte que chaque source lumineuse et/ou chaque appareil de capture d'image du support de mesure est disposé du côté des cercles ou des lentilles ophtalmiques opposé à chaque branche de la monture de vision.

Dans ce cas, les sources lumineuses et/ou les appareils de capture d'image sont disposés à l'extérieur de la monture de vision, de telle sorte que les cercles de la monture et/ou les lentilles ophtalmiques sont situées entre les yeux du porteur et les sources lumineuses et appareils de capture d'image du dispositif de suivi des directions de regard lorsque la monture de vision est placée sur le visage du porteur.

Quel que soit le mode de réalisation envisagé, les sources lumineuses 110 ; 210 ; 310 sont avantageusement des sources lumineuses émettant dans les longueurs d'ondes infra-rouge. La lumière émise n'est alors pas visible par le porteur, et ne perturbe pas sa posture et son comportement visuel. En revanche, cette lumière infra-rouge peut être détectée par le capteur des appareils de capture d'image correspondants.

Ces appareils de capture d'image sont de préférence des caméras adaptées à détecter la lumière émise dans les longueurs d'onde infra-rouge.

De manière avantageuse, le support de mesure 150 ; 250 ; 350 comporte également une licorne 155 ; 255 ; 355, s'étendant perpendiculairement à la barre principale 151 ; 251 ; 351, dans un plan sensiblement perpendiculaire au plan moyen des cercles 411, 412 de la monture 410, ou des lentilles ophtalmiques dans le cas d'une monture percée, lorsque le support de mesure 150 ; 250 ; 350 est fixé sur cette monture 410, et un élément en saillie 156; 256; 356 s'élevant perpendiculairement à la barre principale 151 ; 251 ; 351 et à la licorne 155 ; 255 ; 355, dans le plan moyen des cercles 411, 412 de la monture 410 ou dans un plan parallèle à ce plan moyen lorsque le support de mesure 150 ; 250 ; 350 est fixé sur cette monture 410.

Ici, le support de mesure 150, 250, 350 comporte en outre trois ou quatre éléments de repère R1 ; R2. Ces éléments de repère ne font pas l'objet de l'invention et seules quelques unes de leurs caractéristiques seront rappelées ici.

Un ou deux premiers éléments de repère R1 ; R2 sont disposés à l'une des extrémités de la barre principale 151 ou à chacune de ces extrémités, et sont orientés de manière à être visibles sur une image de face du porteur, lorsque le support de mesure 150 ; 250 ; 350 est fixé sur la monture 410 du porteur.

Un deuxième élément de repère R1 ; R2 est disposé sur l'élément en saillie 156 ; 256 et un troisième élément de repère R1 ; R2 est disposé à l'extrémité de la licorne 155 ; 255, de telle sorte que ces deux éléments de repère sont visibles sur une image de face du porteur.

En outre, les deuxième et troisième éléments de repère R1 ; R2 sont disposés de telle sorte que, sur une image de face du support de mesure 150 ; 250 ; 350, ils sont situés l'un en dessous de l'autre.

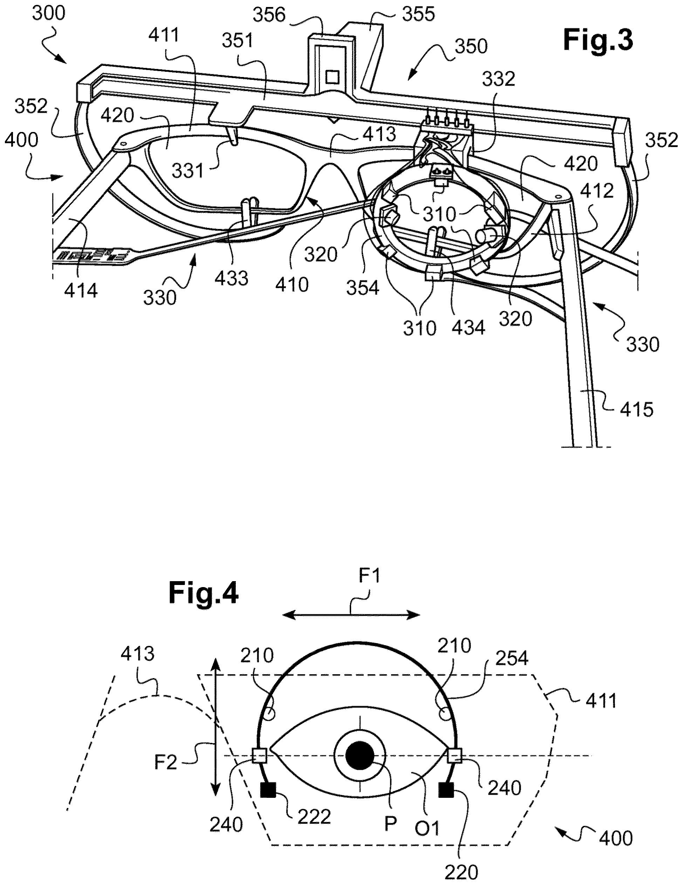

Les éléments de repère du troisième mode de réalisation du support de mesure 350 ne sont pas visibles sur la figure 3.

Chaque élément de repère R1 ; R2 présente une ou plusieurs caractéristiques géométriques prédéterminées, par exemple ses dimensions ou les dimensions d'un motif géométrique porté par lui. Le motif géométrique peut par exemple se présenter sous la forme d'une mire ou de bandes contrastées alternées.

De manière générale, différentes configurations peuvent être envisagées pour le nombre et le positionnement des sources lumineuses et des appareils de capture d'image.

On peut prévoir par exemple au moins deux sources lumineuses et deux appareils de capture d'image, agencés de telle sorte qu'au moins l'une des deux sources lumineuses et l'un des deux appareils de capture d'images sont disposés en vis-à-vis de chaque cercle ou de chaque lentille ophtalmique de la paire de lunette lorsque les moyens de montage du support de mesure coopèrent avec ladite paire de lunettes.

C'est le cas dans les premier et deuxième modes de réalisation du dispositif de suivi de la direction de regard 100 ; 200 représenté sur les figures 1 et 2.

On a alors, pour chaque oeil du porteur, au moins une source lumineuse et un appareil de capture d'image disposé en correspondance.

Ceci permet avantageusement de réaliser des acquisitions de données pour les deux yeux du porteur de manière simultanée. L'encombrement est réduit et l'obstruction du champ de vision du porteur lié à la présence de ces éléments est limitée.

On utilise de préférence une source lumineuse peu directive, adaptée à éclairer la plus grande partie possible de l'oeil du porteur, et un appareil de capture d'image avec un grand angle, c'est-à-dire avec une distance focale faible.

Cependant, on prévoit de préférence, pour chaque oeil du porteur, au moins une source lumineuse, permettant au moins d'éclairer cet oeil du porteur, et au moins deux appareils de capture d'image permettant d'enregistrer des images stéréoscopiques de cet oeil du porteur.

En outre, de préférence, on prévoit deux sources lumineuses en correspondance de chaque oeil du porteur, ce qui autorisera une recherche des reflets cornéens de ces deux sources lumineuses sur les images enregistrées, comme expliqué ultérieurement.

Plus précisément, dans le premier mode de réalisation du dispositif de suivi de la direction de regard 100 représenté sur la figure 1, il est prévu deux sources lumineuse 110 et deux caméras 210 disposées à proximité de chaque cercle 411, 412 de la monture 410.

Chaque source lumineuse 110 et chaque appareil de capture d'image 210 est porté par l'un des bras latéraux 152 du support de mesure 150.

Chaque source lumineuse 110 est ici associée à une caméra 210 dans un élément de mesure 130A, 130B, 130C, 130D.

Chaque élément de mesure 130A, 130B, 130C, 130D comprend ainsi une enveloppe logeant l'une des sources lumineuse 110 et l'une des caméras 210.

Chaque élément de mesure 130A, 130B, 130C, 130D comprend également des moyens d'accrochage de l'enveloppe sur l'un des bras latéraux 152 du support de mesure 150.

Ces moyens d'accrochage peuvent par exemple inclure un logement dans lequel passe le bras latéral 152 correspondant du support de mesure 150 et une vis venant serrer ce bras latéral 152.

Il peut également s'agir de tout autre moyen d'accrochage, par exemple par vissage, collage, clipsage, emboîtement.

Comme représenté sur la figure 1, les éléments de mesure 130A, 130B, 130C, 130D sont répartis de manière à ce que le champ des deux caméras 210 placées à proximité de chaque oeil du porteur couvre la plus grande partie possible de l'oeil du porteur, de préférence au moins une partie comprenant la pupille de cet oeil.

En pratique, sur chaque bras latéral 152 du support de mesure 150, l'un 130B, 130C des éléments de mesure est disposé à proximité de l'extrémité de ce bras latéral 152 comportant les moyens de montage 133, 134 du support de mesure 150 sur la paire de lunettes 400 du porteur et l'autre 130A, 130D des éléments de mesure est disposé à peu près au milieu du bras latéral 152.

Un tel support de mesure présente l'avantage de s'adapter facilement à tous les types de montures de lunettes : cerclées en plastique, en métal, percées, semi-cerclées...quelle que soit la taille de la monture.

En outre, le placement des éléments de mesure est peu gênant pour la vision du porteur. Le champ de vision de celui-ci est peu perturbé.

Ce support de mesure 150 est de préférence réalisé dans un matériau peu dense de manière à ne pas excessivement alourdir la paire de lunettes du porteur.

Dans le deuxième mode de réalisation du dispositif de suivi de la direction de regard 200 représenté sur la figure 2, il est prévu, à titre d'illustration, une configuration de sources lumineuses et de caméras différentes pour chaque oeil du porteur.

En effet, le support de mesure 250 comprend ici, d'un côté deux caméras 220 et six sources lumineuses 210, adaptées à venir en regard de l'oeil droit du porteur, et de l'autre côté, deux caméras 220 et deux sources lumineuses 210 adaptées à venir en regard de l'oeil gauche du porteur.

Plus précisément, le support de mesure 250 comprend ici deux anneaux de support 253, 254 montés sur ladite barre horizontale 151 du support de mesure 150.

Un premier anneau de support 253 est adapté à venir en regard de l'oeil droit du porteur, et le deuxième anneau de support 254 est adapté à venir en regard de l'oeil gauche du porteur.

Chaque anneau de support 253, 254 support l'ensemble des caméras 220 et des sources lumineuses 210 adaptés à venir en regard de l'oeil correspondant.

Chaque anneau de support 253, 254 est fixé sur un montant vertical 255, 256 monté sur une barre secondaire 257.

La barre secondaire 257 s'étend parallèlement à la barre principale 251 du support de mesure 250. Elle est montée sur une autre partie du support de mesure 250, ici sur la licorne 255 du support de mesure 250.

En outre, de préférence, les montants verticaux 255, 256 sont chacun montés mobile en translation sur la barre secondaire 257, le long de celle-ci et perpendiculairement à celle-ci. Ceci peut par exemple être réalisé grâce à une liaison glissière et une crémaillère. Les montants verticaux sont alors mobiles en translation de manière indépendante entre les yeux du porteur.

Les montants verticaux 255, 256 sont mobiles en translation selon un axe parallèle à la droite reliant des deux clips 231, 232 de la barre horizontale 251 et/ou mobile en translation selon un axe perpendiculaire à cette droite.

Ici la droite reliant des deux clips 231, 232 de la barre horizontale 251 est parallèle à la barre principale 251 du support de mesure 250.

Les montants verticaux 255, 256 sont ici mobiles en translation parallèlement à cette barre principale 251 et perpendiculairement à celle-ci, dans un plan sensiblement perpendiculaire à la licorne 255. Ce faisant, en position d'utilisation du support de mesure, les anneaux de support 253, 254 sont mobiles en translation selon deux directions orthogonales d'un plan sensiblement parallèle au plan moyen des cercles de la monture ou des lentilles ophtalmiques.

Ces deux degrés de liberté autorisent le positionnement précis de chaque anneau de support 255, 256 devant l'oeil du porteur, et même un centrage de l'anneau de support sur la pupille P de l'oeil du porteur correspondante, représentée schématiquement sur la figure 2.

En variante, la barre secondaire peut être montée mobile en translation parallèlement à la barre principale du support de mesure. Ceci peut par exemple être réalisé à l'aide d'une glissière ménagée dans la licorne et dans laquelle la barre secondaire coulisse.

On peut également envisager que les anneaux de support soient mobiles en translation selon une troisième direction orthogonale aux deux premières directions de mobilité décrites ci-dessus. Cela permet alors de modifier la distance entre les anneaux de support et les yeux du porteur.

En outre, on peut également envisager des mobilités en rotation autour des montants verticaux et autour de deux axes perpendiculaires entre eux et perpendiculaires au montant vertical correspondant. Des mobilités incluant des mouvements en translation et/ou des pivotements en combinaison peuvent également être envisagées.

En variante, on peut également envisager que les anneaux de support restent fixes, non mobiles par rapport à la barre principale du support de mesure.

Dans le cas où les sources lumineuses et les appareils de capture d'image sont disposés à l'extérieur de la paire de lunettes, de telles mobilités en rotation permettent de tenir compte des effets prismatiques introduits par la présence de la lentille ophtalmique entre l'oeil du porteur et les appareils de capture d'image et sources lumineuses du dispositif de suivi de la direction de regard.

Le premier anneau de support 253 est un anneau fermé, tandis que le deuxième anneau de support 254 est un anneau ouvert.

L'utilisation du premier anneau de support 253 fermé a pour avantage de permettre l'agencement d'un plus grand nombre de caméras et de sources lumineuses sur cet anneau. L'utilisation du deuxième anneau de support 254 ouvert a pour avantage de limiter l'étendue du champ de vision du porteur obstrué par le support de mesure.

De manière générale, chaque anneau de support 253, 254 est en outre réalisé de préférence dans un matériau transparent et présente une épaisseur radiale et axiale la plus fine possible. En outre, le diamètre de l'anneau de support 253, 254 est choisi de manière à préserver le champ de vision du porteur.

Le diamètre intérieur de l'anneau de support est par exemple compris entre 2 et 4 centimètres.

Chaque anneau de support 253, 254 peut en outre comporter un ou plusieurs repères de position 240 (non représenté sur la figure 2, mais visible sur la figure 4) autorisant un réglage prédéterminé de la position des appareils de capture d'image et des sources lumineuses par rapport aux yeux et notamment aux pupilles P du porteur. Ainsi, la position relative de chaque sources lumineuses 210 et de chaque appareil de capture d'image 220 étant connue et fixée par rapport au repère de position sur l'anneau de support 253, 254, le positionnement de ces repères de position 240 par rapport à la pupille P du porteur permet de placer les sources lumineuses 210 et les appareils de capture d'image 220 à une position prédéterminée et connue par rapport à la pupille P du porteur.

Ce réglage de position sera décrit ultérieurement.

Ici, comme évoqué précédemment, six sources lumineuses et deux caméras sont supportées par le premier anneau de support 253, tandis que deux sources lumineuses et deux caméras sont supportées par le deuxième anneau de support 254.

Ces sources lumineuses et caméras sont fixées sur l'anneau de support correspondant par tout moyen connu de l'homme du métier, par exemple par vissage, collage, clipsage, emboîtement.

Comme représenté sur la figure 2, les sources lumineuses 210 et les caméras 220 sont répartis autour de chaque anneau de support 253, 254 de manière à ce que le champ des deux caméras 220 placées à proximité de chaque oeil du porteur couvre la plus grande partie possible de l'oeil du porteur, de préférence au moins une partie comprenant la pupille de cet oeil, et de manière à pouvoir suivre la position de la pupille de l'oeil dans le plus grand nombre de direction de regard possible.

Sur les deux anneaux de support 253, 254, les deux caméras sont écartées d'un angle au centre d'environ 120 degrés, symétriques par rapport à un plan passant par un diamètre de l'anneau de support et perpendiculaire à la droite reliant les clips de montage 231, 232 de la barre principale 251 du support de mesure 250.

Les sources lumineuses 210 sont réparties régulièrement et deux à deux diamétralement opposées.

Ce support de mesure 250 présente l'avantage de garantir un positionnement précis des sources lumineuses et caméras indépendant de la forme des montures choisies par le porteur.

Comme dans le cas du troisième mode de réalisation de la figure 3, on peut également prévoir que les sources lumineuses 310 et les appareils de capture d'image 320 du support de mesure 350 sont agencés de telle sorte qu'ils sont disposés en vis-à-vis d'un unique cercle 412 ou d'une unique lentille ophtalmique de la paire de lunettes 400 lorsque les moyens de montage 330 du support de mesure 350 coopèrent avec ladite paire de lunettes.

Plus précisément, le support de mesure 350 du troisième mode de réalisation comporte ici un unique anneau de support 353 supportant six sources lumineuses 310 et deux caméras 320.

De manière générale, on peut envisager au moins une source lumineuse et au moins un appareil de capture d'image adaptés à être disposé en regard d'un même cercle de la monture ou d'une même lentille ophtalmique de la paire de lunettes.

L'anneau de support 353 est tout à fait semblable au premier anneau de support 253 du deuxième mode de réalisation décrit précédemment et ne sera pas décrit plus en détails ici.

L'anneau de support 353 est ici fixé sur la barre principale 351 du support de mesure 350, au niveau de l'un des clips 332 de fixation de la barre principale 351 sur la paire de lunettes 400. Il est ici fixé sans mobilité sur une languette rigide qui s'étend à partir de la barre principale 351.

Le suivi de la direction de regard est ici monoculaire.

Quel que soit le mode de réalisation considéré, le dispositif de suivi de la direction du regard obtenu est un dispositif miniature, autonome et nomade.

Les appareils de capture d'image 120 ; 220 ; 320 du support de mesure communiquent avec les moyens de détermination de la position du point remarquable de l'oeil du porteur, donc avec les moyens de détermination de la direction de regard du porteur, pour leur transmettre les images enregistrées.

Les moyens de détermination de la position du point remarquable de l'oeil du porteur, donc les moyens de détermination de la direction de regard du porteur, peuvent également communiquer avec les sources lumineuses, par exemple de manière à commander leur allumage/extinction.

A cet effet, le support de mesure comprend une interface de communication entre les sources lumineuses et/ou les appareils de capture d'image et les moyens de détermination de la position du point remarquable de l'oeil du porteur et/ou de la direction de regard. Il s'agit de toute interface de communication connue de l'homme du métier. Cette interface de communication peut être filaire ou sans fil.

Elle échange des données avec un poste distant, notamment pour une phase d'initialisation et une phase finale de récupération des données enregistrées. Ces phases seront décrites ultérieurement.

En pratique, les moyens de détermination de la position du point remarquable de l'oeil du porteur et les moyens de détermination de la direction du regard comprennent de manière générale des moyens informatiques déportés programmés pour mettre en oeuvre respectivement l'un des modes de réalisation du procédé de détermination de la position du point remarquable de l'oeil du porteur et de la direction de regard selon l'invention décrit plus loin. Ces moyens de détermination sont ici regroupés dans un unique terminal informatique.

De manière optionnelle, le dispositif de détermination de la position d'un point remarquable de l'oeil du porteur, et donc le dispositif de suivi de la direction de regard, peut également comprendre d'autres éléments le rendant multi-fonction.

Il peut comporter notamment une ou plusieurs caméras de scène, c'est-à-dire tournées à l'opposé du porteur de manière à capturer des images de son environnement. Cela est notamment utile pour la classification des activités du porteur, l'enrichissement de la carte de disparités de distances, l'évaluation de la carte de luminosité de l'environnement du porteur. Le comportement visuel du porteur peut ainsi être mis en relation avec les différentes activités du porteur. Le comportement oeil-tête, les postures et les déplacements du porteur sont ainsi plus précisément déterminés.

Il peut comporter également des capteurs annexes, notamment des capteurs miniatures du type accéléromètre, gyroscope, magnétomètre, des capteurs de luminosité, des capteurs de distance et de proximité, des capteurs de géolocalisation.

Il peut enfin comprendre des moyens d'interaction entre le porteur et le dispositif de suivi de la direction de regard 100 ; 200 ; 300, notamment via des boutons et autres capteurs haptiques, et des dispositifs pour le retour d'informations du type diode électroluminescente, écrans miniatures, affichage tête-haute, vibreur, synthèse vocale, ...

En variante, on peut envisager que les moyens de positionnement sont adaptés à coopérer avec la paire de lunettes de l'individu pour positionner seulement chaque appareil de capture d'image. Ils possèdent alors éventuellement des moyens additionnels pour positionner la ou les sources lumineuses.

Dans une telle variante, les sources lumineuses du dispositif de suivi de la direction du regard peuvent être déportées.

En d'autres termes, les sources lumineuses peuvent être positionnées ailleurs que sur la paire de lunettes du porteur, à distance de celle-ci. On peut par exemple envisager que les sources lumineuses sont fixées sur un mur faisant face au porteur, grâce à différents systèmes de fixation préparés dans le mur. Le porteur est par exemple positionné à un endroit donné matérialisé par un marquage au sol. Le marquage au sol et les systèmes de fixation des sources lumineuses sur le mur sont agencés de manière à ce que chaque source lumineuse éclaire au moins l'un des yeux du porteur. Ils font alors partie des moyens de positionnement du dispositif de suivi de la direction du regard.

Enfin, on peut également envisager que le rôle de ces sources lumineuses soit joué par la lumière ambiante, que ce soit le soleil ou la lumière artificielle ambiante. La ou les sources lumineuses peuvent dans ce cas ne pas être inclues dans le dispositif de détermination de la position d'un point remarquable de l'oeil et donc dans le dispositif de suivi de la direction de regard.

Selon une autre variante du dispositif selon l'invention, on peut envisager que le support de mesure comporte en outre au moins un guide d'onde acheminant la lumière émise par une source lumineuse.

On peut ainsi envisager que les sources lumineuses se trouvant déportées hors du champ de vision du porteur, leur lumière est acheminée jusqu'à l'oeil du porteur par un tel guide d'onde.

Avantageusement, on peut envisager que l'anneau de support des deuxième et troisième modes de réalisation forme lui-même un guide d'onde. La source lumineuse reliée à ce guide d'onde peut-être positionnée à distance de la tête du porteur ou bien placée sur le support de mesure, sur la barre principale ou l'un des bras latéraux.

En variante, le guide d'onde peut également être placé du côté de la monture de vision opposé à celui où s'étend la ou les branches de cette monture.

Le guide d'onde peut notamment être placé à proximité de la face avant des lentilles ophtalmiques de la monture de vision.

Le guide d'onde peut également comprendre une ou plusieurs fibres optiques. Une telle variante présente l'avantage de limiter l'obstruction du champ de vision du porteur.

De nombreuses variantes peuvent être envisagées pour le dispositif décrit précédemment.

On peut par exemple envisager un support de mesure comportant au moins un anneau de support semblable à celui décrit précédemment, muni de moyens de montage adaptés à un montage direct de cet anneau sur la paire de lunettes, par exemple au moyen d'une ventouse autorisant l'accrochage de l'anneau de support sur une lentille ophtalmique de la paire de lunettes.

On peut également envisager que l'anneau de support soit remplacé par une structure filiforme présentant une forme quelconque adaptée à préserver le champ de vision du porteur, faite par exemple d'un fil de préférence transparent, qui peut être ou non flexible.

Il est également possible d'envisager que le support de mesure comprenne une lentille plane présentant une forme proche de celle des cercles de la monture, sur laquelle sont montés un anneau de support et/ou les appareils de capture d'image et /ou les sources lumineuses, la lentille plano afocale pouvant être fixée sur la monture par tout moyen connu de l'homme du métier, par exemple par un système de fixation comportant des clips et/ou des aimants.

### Procédé

Le dispositif de suivi de la direction du regard 100 ; 200; 300 décrit précédemment est utilisé pour la conception optique personnalisée de lentilles ophtalmiques adaptées à la fois au porteur et à la monture choisie.

En particulier, il est utilisé pour déterminer la position et l'étendue des zones d'usure de chaque lentille ophtalmique destinée au porteur et à la monture choisie. Pour cela, les directions de regard du porteur, puis les points d'intersection de ces directions de regard du porteur avec un plan lié à la monture ou à la lentille ophtalmique considérée, sont déterminées pendant que le porteur effectue différentes tâches visuelles alors qu'il est équipé du dispositif de suivi de la direction de regard décrit précédemment.

De manière générale, pour la détermination de la direction de regard d'au moins un oeil O1 du porteur équipé de la paire de lunettes 400, au moyen du dispositif pour le suivi de la direction de regard 100 ; 200 ; 300 qui comporte au moins une source lumineuse 110 ; 210 ; 310 et au moins un dispositif de capture d'image 210 ; 220 ; 320, l'opérateur réalise les étapes suivantes :
a) positionner le dispositif pour le suivi de la direction de regard 100 ; 200 ; 300 comprenant le dispositif pour la détermination d'un point remarquable de l'oeil par rapport à la paire de lunettes 400 du porteur,
c) capturer au moins une image de l'oeil du porteur avec l'appareil de capture d'image 120 ; 220 ; 320 dudit dispositif de suivi 100 ; 200 ; 300,
d) déterminer la position d'un point remarquable de l'oeil à partir de l'image capturée,
e) déterminer la direction de regard de l'individu en fonction de la position de ce point remarquable de l'oeil.

De préférence, le dispositif de suivi 100 ; 200 ; 300 comprend au moins une source lumineuse 110 ; 210 ; 310.

Le procédé de détermination de la direction de regard comprend alors en outre une étape b) d'illumination de l'oeil O1 du porteur grâce à la source lumineuse 110 ; 210 ; 310 dudit dispositif de suivi 100 ; 200 ; 300.

Les étapes a), b), c), d) constituent un procédé de détermination de la position d'un point remarquable de l'oeil.

Nous expliciterons dans la suite les étapes de ce procédé de suivi de la direction de regard en relation avec les modes de réalisation du dispositif de suivi de la direction du regard décrit précédemment.

Ce procédé de suivi de la direction de regard repose sur un protocole de mesure réalisable en magasin par l'opticien. La position et le champ des appareils de capture d'image sont prédéterminés en usine à la fabrication du dispositif de suivi de la direction de regard 100 ; 200 ; 300. Seule une étape de réglage de la position verticale et/ou horizontale et éventuellement de l'orientation des appareils de capture d'image est nécessaire, comme expliqué ci-dessous. Le procédé est donc simple et rapide à mettre en oeuvre.

Plus précisément, à l'étape a), l'opérateur positionne le dispositif pour le suivi de la direction de regard en plaçant le support de mesure 150 ; 250 ; 350 sur la paire de lunettes 400 du porteur. Ce positionnement implique celui du dispositif de détermination de la position d'un point remarquable de l'oeil. Il peut s'agir d'une ancienne paire de lunettes ou d'une nouvelle monture choisie, qui comporte de préférence des lentilles ophtalmiques correctrices ou de présentation. Cela est ici réalisé en pinçant la monture 410 ou les lentilles ophtalmiques 420 de la paire de lunettes 400 entre les pions des clips 131, 132, 133, 134 ; 231, 232, 233, 234 ; 331, 332, 433 de montage du dispositif de suivi de la direction du regard 100 ; 200 ; 300.

Ce positionnement est réalisé de manière à assurer que la ou les sources lumineuses 110 ; 210 ; 310 du dispositif éclairent l'oeil du porteur, que le ou les appareils de capture d'image 120 ; 220 ; 320 capture une image d'au moins une partie de l'oeil, et de manière à ce que le champ de vision du porteur soit le moins possible perturbé par la présence de ces éléments.

Dans le cas du premier mode de réalisation, la position des sources lumineuses 110 et des appareils de capture d'image 120 est étroitement liée à la forme générale de la monture 410 et/ou des lentilles ophtalmiques 420. Il est possible cependant d'ajuster empiriquement la position relative des sources lumineuses et des appareils de capture d'image par rapport aux yeux du porteur, par exemple en ajustant l'inclinaison de chaque élément de mesure 130A, 130B, 130C, 130D sur le bras latéral 152 du support de mesure 150 lorsque les moyens d'accrochage de ces éléments de mesure sur le bras latéral le permettent.

Dans le cas du deuxième mode de réalisation du dispositif selon l'invention, la position de ce dispositif de suivi de la direction de regard par rapport aux yeux du porteur est ajustée de manière à centrer les sources lumineuses 210 et les appareils de capture d'image 220 sur la pupille P de l'oeil lorsque le porteur regarde droit devant lui.

De préférence, cet ajustement permet de déterminer la position relative des sources lumineuses et des appareils de capture d'image par rapport aux yeux du porteur.

Pour cela, l'opérateur demande au porteur de regarder dans une direction de regard spécifique. Le point d'intersection entre cette direction de regard et au moins l'une des lentilles ophtalmiques 420 de la paire de lunettes 400 est déterminé. Il peut s'agir par exemple d'un point d'intersection prédéterminé.

La position du point d'intersection prédéterminé peut être notée ou non sur la lentille ophtalmique. La position de ce point d'intersection prédéterminé peut aussi être mesurée par un autre équipement.

L'opérateur ajuste la position du ou des appareils de capture d'image 120 ; 220 par rapport à la position de ce point d'intersection.

En pratique, l'opérateur peut par exemple demander au porteur de regarder au loin, à l'horizon. Dans ce cas, la position du point d'intersection est celle de la croix de montage de la lentille ophtalmique.

Ce cas est représenté sur la figure 4.

L'opérateur aligne alors les repères de position 240 du support de mesure 250 avec la pupille P de l'oeil du porteur pendant que celui-ci regarde au loin, grâce aux mobilités en translation de l'anneau de support 254 disposé en regard de l'oeil gauche du porteur, représentées par les flèches F1 et F2 de la figure 4.

Un réglage similaire peut être réalisé pour positionner l'anneau de support 253 disposé en regard de l'oeil droit du porteur dans le deuxième mode de réalisation.

Cela revient à aligner le centre de l'anneau de support 253, 254 avec le centre de la pupille P dans le cas des deuxième et troisième modes de réalisation du dispositif de suivi de la direction de regard. Ce réglage est représenté par les flèches F1 et F2 de la figure 4.

En outre, on peut également envisager que l'opérateur ajuste l'orientation de chaque anneau de support par rapport à l'oeil du porteur correspondant grâce aux mobilités en rotation autour des montants verticaux 255, 256 et autour de deux axes perpendiculaires entre eux et perpendiculaires au montant vertical correspondant.

Dans le cas du troisième mode de réalisation, l'anneau de support 353 ne présente pas de mobilité par rapport au support de mesure. C'est donc l'ensemble de ce support de mesure 350 qui peut être légèrement déplacé par l'opérateur afin de centrer correctement l'anneau de support 353 sur l'oeil du porteur.

Les appareils de capture d'image 120 ; 220; 320 ainsi positionnés permettent de suivre le déplacement des pupilles P dans toutes les directions.

Afin d'assurer un positionnement correct des appareils de capture d'image, quel que soit le mode de réalisation envisagé, il est possible de prévoir une étape de visualisation par l'utilisateur d'une image obtenue par les appareils de capture d'image de manière à vérifier qu'au moins un oeil du porteur est bien visible sur cette image. Le réglage de la position des appareils de capture d'image est ainsi facilité.

Pendant la réalisation des étapes b) et c), l'opérateur demande par exemple au porteur de suivre un protocole permettant de favoriser les visions de loin et/ou la vision à distance intermédiaire et/ou la vision de près du porteur.

On parle par exemple de vision de près pour des tâches visuelles imposant de fixer son regard sur un point situé entre 20 et 40 centimètres du porteur.

On parle de vision intermédiaire pour des tâches visuelles imposant de fixer son regard sur un point situé environ entre 40 centimètres et 4 mètres du porteur.

On parle par exemple de vision de loin pour des tâches visuelles imposant de fixer son regard sur un point situé au-delà d'environ 4 mètres du porteur.

Par exemple, l'opérateur demande au porteur d'avoir une activité de marche pour favoriser la vision de loin, et/ou une activité de lecture pour favoriser la vision de près et/ou une activité de travail sur ordinateur pour favoriser la vision à distance intermédiaire.

Les appareils de capture d'image 120; 220; 320 et les sources lumineuses 110 ; 210 ; 310 sont activés lors de ces activités, et, à l'étape e), les directions de regard sont déterminées par le dispositif de suivi de la direction de regard en correspondance avec chaque activité.

Plus précisément, à l'étape b), on commande l'émission de lumière par les sources lumineuses 110 ; 210 ; 310 ou on s'assure que la lumière émise par les sources lumineuses déportées ou ambiante illumine bien l'oeil du porteur. Cette commande peut être envoyée à distance ou donnée manuellement par l'enfoncement d'un bouton.

A l'étape c), on commande la capture d'au moins une image par ledit au moins un appareil de capture d'image.

Les différents modes de réalisations du dispositif de suivi de la direction de regard décrit précédemment comprennent au moins deux appareils de capture d'image adaptés à être disposés en vis-à-vis de chaque oeil. A l'étape c) on capture alors une image grâce à chaque appareil de capture d'image 120 ; 220 ; 320, simultanément. Une image stéréoscopique de l'oeil est donc obtenue.

Les données relatives aux images capturées sont transmises par un réseau sans fil wifi au terminal informatique.

A l'étape d), le traitement de ces images par le terminal informatique permet donc de déterminer les coordonnées en trois dimensions du point remarquable de l'oeil O1 du porteur que l'on identifie sur les images capturées à l'étape c) par chaque appareil de capture d'image. Ces coordonnées sont par exemple déterminées dans un référentiel lié aux appareils de capture d'image.

On détermine par exemple la position de ce point remarquable de l'oeil O1 par un calcul de triangulation à partir de ces deux images capturées par les deux dispositifs de capture d'image 120 ; 220 ; 320.

Le point remarquable de l'oeil dont on détermine la position à l'étape d) est de préférence un élément de structure de l'oeil pouvant être le centre de la pupille P de l'oeil, un point relatif à l'iris de l'oeil, un point du contour de l'iris, un point du contour de la pupille, un point relatif à un vaisseau sanguin de l'oeil, ou encore le canthus externe ou interne de l'oeil.

Il s'agit, dans les exemples développés ici du centre de la pupille P de l'oeil.

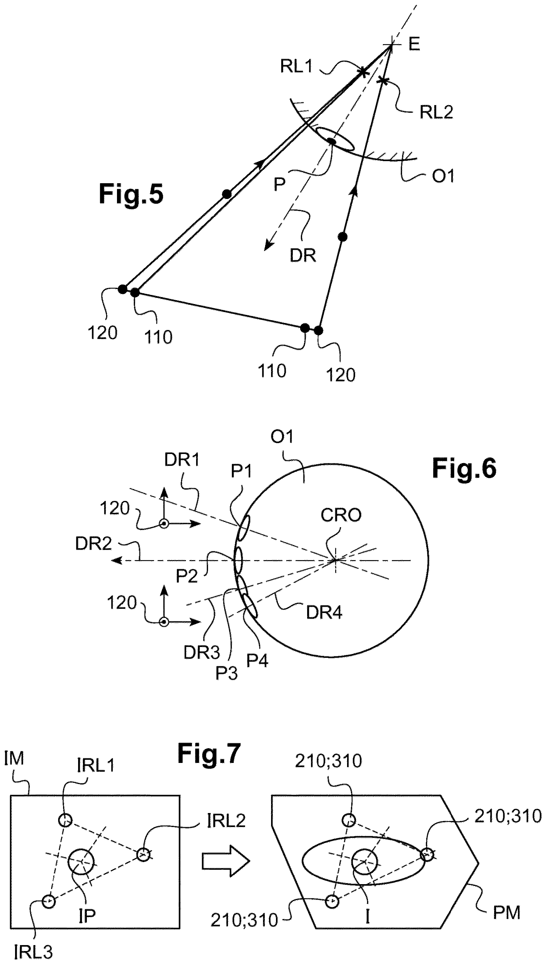

Selon un premier mode de réalisation du procédé de détermination de la direction du regard, en référence à la figure 5, le dispositif de suivi de la direction de regard comprend en outre une source lumineuse associée à chaque appareil de capture d'image.

Plus précisément, le dispositif de suivi de la direction du regard comprend alors, en regard de chaque oeil du porteur, deux sources lumineuses 110 et deux appareils de capture d'image 120 dont les positions et orientations sont connues dans le plan moyen des cercles 411 ; 412 de la monture 410 ou des lentilles ophtalmiques 420 montées dans la monture 410. Chaque source lumineuse 110 est positionnée à proximité de l'un des deux appareils de capture d'image 120 correspondant. Cette configuration correspond par exemple à l'utilisation du premier mode de réalisation du dispositif de suivi de la direction du regard 100, représenté à la figure 1, dans lequel les éléments de mesure 130A, 130B, 130C, 130D regroupent chacun une source lumineuse 110 et un appareil de capture d'image 120.

Alors, à l'étape e), pour l'un des yeux du porteur,
- on identifie sur chaque image capturée les images des reflets cornéens RL1, RL2 des deux sources lumineuses 110,
- on détermine la position en trois dimensions des reflets cornéens RL1, RL2 des deux sources lumineuses 110 par des calculs de triangulation à partir de ces deux images capturées par les deux dispositifs de capture d'image 120,
- on détermine la position en trois dimensions du centre E de la courbure de la cornée de l'oeil O1 assimilée à une sphère en fonction de la position des reflets cornéens RL1, RL2 des sources lumineuses 110,
- on en déduit la direction de regard DR comme étant la droite reliant ce centre E de la courbure de la cornée de l'oeil et le centre de la pupille P.

Par exemple, le centre de la courbure de la cornée est le centre de la sphère ayant le rayon de courbure moyen de la cornée au point considéré. Il s'agit en particulier ici du centre de la courbure moyenne.

Plus précisément, pour déterminer la position en trois dimensions du centre E de la courbure de la cornée de l'oeil O1, on détermine la position du point d'intersection entre les deux droites reliant la pupille d'entrée de chaque appareil de capture d'image 120 et le reflet cornéen RL1, RL2 de la source lumineuse 110 associée. On identifie le centre E de la courbure de la cornée de l'oeil à ce point d'intersection.

La direction de regard est alors donnée par la droite reliant le centre de la cornée de l'oeil ou le centre de rotation de l'oeil et le centre de la pupille de l'oeil. Cette direction de regard est par exemple corrigée d'un décalage de quelques degrés, typiquement 2 à 4 degrés, car l'axe visuel est anatomiquement décalé par rapport à la direction de regard.

Selon un deuxième mode de réalisation du procédé de détermination de la direction de regard, en référence à la figure 6, les positions et orientations des appareils de capture d'image 120 ; 220 ; 320 sont connues dans le plan moyen des cercles 411 ; 412 de la monture 410 ou des lentilles ophtalmiques 420 montées dans la monture 410. Les reflets cornéens des sources lumineuses ne sont pas ici identifiés et les sources lumineuses 110 ; 210 ; 310 sont utilisées uniquement pour l'éclairage des yeux du porteur.

Ce deuxième mode de réalisation du procédé peut être mis en oeuvre par les trois modes de réalisation du dispositif de suivi du regard décrit précédemment.

On peut notamment le mettre en oeuvre lorsque l'anneau de support est utilisé comme un guide d'onde et éclaire l'oeil du porteur.

Alors, à l'étape e), pour l'un des yeux du porteur,
- on détermine la position en trois dimensions du centre de rotation CRO de l'oeil O1,
- on en déduit la direction de regard DR1, DR2, DR3, DR4 comme étant la droite reliant ce centre de rotation CRO de l'oeil et la pupille P1, P2, P3, P4 dont la position a été déterminée à l'étape d).

Plus précisément, selon une première variante, pour déterminer la position en trois dimensions du centre de rotation CRO de l'oeil O1, on utilise un modèle de l'oeil dans lequel la position du centre de rotation CRO est déterminée.

Pour déterminer ce modèle de l'oeil, on peut par exemple identifier l'image de la pupille P1, P2, P3, P4 de l'oeil O1 sur une pluralité de couples d'images de l'oeil capturées simultanément par les deux appareils de capture d'image 120 ; 220 ; 320 pour différentes directions de regard du porteur, déterminer la position des pupilles P1, P2, P3, P4 de l'oeil O1 en trois dimensions pour ces différentes directions du regard et déterminer le modèle de l'oeil comme étant une sphère passant par les points ayant les positions des pupilles P1, P2, P3, P4 de l'oeil pour ces différentes directions du regard. La position du centre de rotation CRO est ensuite déterminée comme étant le centre de cette sphère.

Selon une deuxième variante, pour déterminer la position en trois dimensions du centre de rotation CRO de l'oeil O1, on la mesure grâce à un équipement et selon une méthode connue de l'homme du métier, par exemple en utilisant le dispositif « Visioffice » d'Essilor. Cette étape correspond à une étape préalable de calibrage.

Selon un troisième mode de réalisation du procédé de détermination de la direction de regard, en référence à la figure 7, le dispositif pour le suivi de la direction de regard 200 ; 300 comporte au moins trois sources lumineuses 210 ; 310 en vis-à-vis de l'oeil du porteur. C'est le cas des deuxième et troisième modes de réalisation. Les positions de ces sources lumineuses 210 ; 310 par rapport aux appareils de capture d'image sont inconnues initialement.

Elles sont de préférence aussi peu excentrées que possible par rapport à l'oeil du porteur sans que cela perturbe la vision du porteur.

Il n'est pas utile ici de connaître les positions et orientations des appareils de capture d'image.

Dans une première sous-étape, on réalise un apprentissage de la position relative des sources lumineuses 210 ; 310 par rapport à la monture 410 ou par rapport à un point de référence, par exemple le point de référence de la lentille ophtalmique en vision de loin, c'est-à-dire la croix de montage.

A cet effet, la position des sources lumineuses par rapport à la monture 410 ou par rapport à un point de référence, comme la croix de montage de la lentille ophtalmique par exemple, est ajustée, ou une image de l'ensemble de la monture 410 et du dispositif de suivi de la direction de regard 200 ; 300 est capturée.

Il est également possible d'utiliser la capacité du capteur de l'appareil de capture d'image à détecter les infrarouges proches pour détecter instantanément chacune des sources lumineuses en les activant tour à tour.

On calcule la position apparente du centre de rotation CRO de l'oeil et du rayon apparent de l'oeil dans le champ de l'appareil de capture d'image grâce à un modèle de l'oeil du porteur similaire à celui décrit précédemment, c'est-à-dire en identifiant l'oeil à une sphère passante par les positions des pupilles du porteur déterminées à partir de leurs positions identifiées sur différents couples d'images correspondant à des directions de regard différentes.

Ceci peut être réalisé grâce à des captures d'images, en particulier des images stéréoscopiques, pendant une séquence d'apprentissage sur le porteur en lui demandant de tourner les yeux aléatoirement ou en utilisant des séquences d'images acquises lors du protocole permettant de favoriser les visions de loin et/ou la vision à distance intermédiaire et/ou la vision de près du porteur décrit précédemment.

Ensuite, pour un couple d'images simultanées données, on identifie l'image de la pupille IP de l'oeil et on détermine par un calcul de triangulation, la position en trois dimensions du centre de cette pupille.

On identifie les images IRL1, IRL2, IRL3 des reflets cornéens des sources lumineuses 210 ; 310 et du point remarquable de l'oeil, ici l'image du centre de la pupille IP, sur ces images IM (figure 7) et on détermine par un calcul de triangulation, la position en trois dimensions de ces reflets cornéens et du centre de la pupille pour chaque couple d'images capturées.

L'analyse de la position relative de la pupille par rapport au CRO permet d'estimer la position du centre de la cornée. On utilise par exemple un modèle simplifié dans lequel le centre de rotation de l'oeil, le centre de la cornée et le centre de la pupille sont alignés. On connait grâce à des valeurs médianes, ou des valeurs mesurées spécifiquement sur le porteur, les grandeurs suivantes : le rayon de l'oeil, le rayon cornéen et la position réelle de la pupille, différente de la position de son image au travers de la cornée. On peut ainsi en déduire la position apparente du centre de la cornée sur l'image capturée par l'appareil de capture d'image.

Cela permet de calculer les coordonnées sphériques du centre de la pupille et des reflets cornéens dans le référentiel de la cornée, puis le calcul d'un «système barycentrique » liant les coordonnées sphériques du centre de la pupille dans le référentiel de la cornée aux reflets cornéens. La position relative du centre de la pupille et des reflets cornéens est ainsi déterminée.

On reporte ensuite les coordonnées barycentriques de l'image de la pupille de l'oeil par rapport aux reflets cornéens, dans un référentiel lié à la monture ou aux lentilles ophtalmiques, pour déterminer la position du centre de la pupille dans ce dernier référentiel.

Dans un cas simplifié, correspondant en particulier au cas où les angles entre les reflets cornéens et la direction de regard sont petits, par exemple inférieur à 10 degrés d'angle, ce report des coordonnées barycentriques comprend un calcul basé sur une bijection entre les coordonnées de la pupille dans le référentiel de l'appareil de capture d'image et le point d'intersection de la direction de regard avec une surface liée à la lentille ophtalmique dans le référentiel de cette lentille ophtalmique.

Dans un cas plus complexe, il est nécessaire de connaître la position apparente de l'oeil, et en particulier de la surface de la cornée, afin de pouvoir estimer l'écart angulaire réel entre la pupille et les reflets cornéens.

A partir de la position du centre de rotation de l'oeil et de l'identification de l'image de la pupille sur l'image capturée, on détermine les coordonnées polaires du centre de la pupille et des reflets cornéens des sources lumineuses dans le référentiel de la cornée.

Dans le référentiel de la cornée, on détermine des distances réelles entre les reflets cornéens, comme cela est décrit par exemple dans le document de Arantxa Villanueva, Juan J. Cerrolaza et Rafael Cabeza (2008), intitulé « Geometry Issues of Gaze Estimation », dans « Advances in Human Computer Interaction », Shane Pinder (Ed.), ISBN: 978-953-7619-15-2, InTech, DOI: 10.5772/5911.

On déduit la direction du regard du porteur dans le référentiel de la monture ou des lentilles ophtalmiques à partir de la position des sources lumineuses dans ce référentiel et de la position relative du point remarquable et des reflets cornéens.

Cela est réalisé par exemple en déterminant par un calcul d'optimisation le point d'intersection I (figure 7) de la direction du regard sur le plan moyen PM du cercle de la monture ou de la lentille ophtalmique à partir de la position relative du centre de la pupille et des reflets cornéens déterminée précédemment et des coordonnées des sources lumineuses 210, 310 dans le référentiel de la monture ou des lentilles ophtalmiques. La direction de regard est alors la droite reliant la pupille P de l'oeil au point d'intersection déterminé.

Dans le cas où il n'est prévu qu'un seul appareil de capture d'image devant chaque oeil du porteur, on identifie, à l'étape d), l'image du point remarquable sur l'image capturée. Puis, en tenant compte de la position relative connue de l'appareil de capture d'image par rapport à l'oeil du porteur et d'un modèle de cet oeil dans lequel la position du centre de rotation de l'oeil est connu, on détermine la position du point remarquable dans le référentiel de l'appareil de capture d'image et on en déduit la direction du regard du porteur. L'opérateur peut également demander au porteur de fixer une cible, dont la position est par ailleurs déterminée dans le référentiel de l'appareil de capture d'image. La direction de regard est alors déterminée comme la droite reliant le centre de rotation de l'oeil et la cible.

Dans le cas où le point remarquable dont la position est déterminée à l'étape d) n'est pas le centre de la pupille, on peut envisager de déduire la position du centre de la pupille à partir d'un modèle de l'oeil et de la position de ce point remarquable. Les différents modes de réalisation des procédés décrits précédemment sont alors mis en oeuvre de la même façon.

On peut également envisager dans ce cas de passer par une estimation de la déviation angulaire de la direction de regard par rapport à une direction de regard de référence prédéterminée pour laquelle la position du point remarquable de l'oeil est connue.

En effet, connaissant une direction de regard de référence et la position angulaire de référence correspondante du point remarquable associé, on détermine un écart angulaire entre la direction de regard et le point remarquable considéré. On suppose ensuite que cet écart angulaire reste constant,

Par exemple, le point remarquable peut correspondre à un vaisseau sanguin se trouvant à -15 degrés d'angle dans le plan sagittal et 23 degrés d'angle dans le plan tangentiel de la direction de regard. Le suivi en continu de la position de ce point remarquable, et le calcul des coordonnées sphériques de ce point remarquable dans un référentiel lié au centre de rotation de l'oeil permettent de déduire immédiatement la direction de regard en supposant que la position angulaire de ce point remarquable par rapport à la direction de regard reste constante.

Dans le cas où le dispositif de suivi de la direction de regard est agencé de manière à ce que les sources lumineuses et les appareils de capture d'image soient disposés à l'extérieur de la paire de lunettes, c'est-à-dire de telle sorte que la lentille ophtalmique soit disposé entre l'appareil de capture d'image et l'oeil du porteur, on tient compte des effets prismatiques introduits par la présence de cette lentille ophtalmique lors de la détermination des positions des reflets cornéens ou du point remarquable de l'oeil.

Cela est notamment le cas lorsque la ou les sources lumineuses sont déportées, c'est-à-dire ne sont pas montées sur la paire de lunettes avec le ou les appareils de capture d'image.

Il est alors possible de déterminer les directions de regard réelles, non réfractées à travers les lentilles ophtalmiques, à partir de la géométrie de cette lentille et la répartition de puissance de cette lentille. Cela est également possible dans le cas où la lentille ophtalmique est afocale.

Une fois qu'une pluralité de directions du regard du porteur a été déterminée selon l'un des modes de réalisation du procédé de détermination de la direction du regard décrit précédemment,
- on détermine, pour chaque direction du regard DR, son point d'intersection avec une surface prédéterminée relative à la monture et/ou à la lentille ophtalmique du porteur,
- on détermine une zone d'usure de la lentille ophtalmique destinée à équiper le porteur en fonction desdits points d'intersection.

Il est ainsi possible de déterminer au moins une zone d'usure de cette lentille ophtalmique.

En pratique, on détermine par exemple les coordonnées de l'intersection de la direction du regard avec le plan moyen du cercle 411, 412 correspondant de la monture 410 ou avec la surface moyenne de la lentille ophtalmique 420 correspondante.

La surface moyenne de la lentille ophtalmique est définie comme la surface équidistante en tout point des faces avant et arrière de la lentille.

La surface prédéterminée prise en compte peut également être l'une des faces avant ou arrière de la lentille ophtalmique. Il peut s'agir du plan moyen de la lentille ophtalmique, défini comme le plan ayant les points statistiquement les plus proches de la surface moyenne de cette lentille ophtalmique.

Dans le cas du troisième mode de réalisation du procédé de détermination de la direction de regard, il n'est pas nécessaire de déterminer les coordonnées de cette intersection puisque le point d'intersection I (figure 7) de la direction du regard sur le plan moyen PM du cercle de la monture ou de la lentille ophtalmique est déjà déterminé à partir de la position relative du centre de la pupille et des reflets cornéens déterminée précédemment et des coordonnées des sources lumineuses 210, 310 dans le référentiel de la monture ou des lentilles ophtalmiques, par exemple par un calcul d'optimisation. Les points de la zone d'usure peuvent alors être directement identifiés aux points d'intersection déterminés. Ils peuvent également être déduits de ces points d'intersection en tenant compte d'une correction traduisant le fait que les sources lumineuses ne sont pas, dans un cas général, positionnées dans un plan parallèle au plan moyen du cercle de la monture ou de la lentille ophtalmique considéré.

Le terminal informatique utilise ces informations pour concevoir les nouvelles lentilles ophtalmiques destinées au porteur.

Il s'agit par exemple de déterminer la position et l'étendue des zones d'usure de chaque lentille ophtalmique correspondant à la vision de près, à la vision à distance intermédiaire ou à la vision de loin. Il s'agit alors de cartographier les zones de la lentille ophtalmique utilisées par le porteur en vision de près et celle utilisées en vision de loin.

Ces zones d'usure sont déterminées en fonction de la position des points d'intersection avec le plan prédéterminé des directions de regard déterminées à partir des images capturées pendant les tâches visuelles assurant une vision de près, à distance intermédiaire ou de loin du porteur.

Une zone d'usure peut être déterminée de manière à englober tous les points d'intersection déterminés correspondant à la tâche visuelle concernée.

Cette zone d'usure peut présenter une forme prédéfinie. Elle peut s'étendre de manière à ce que son contour passe à une distance seuil prédéterminée de chaque point d'intersection.

Il peut s'agir d'une ellipse ou d'un rectangle qui contient tout ou un pourcentage des points d'intersection déterminés. De préférence, le contour de la zone d'usure entoure au moins 95% des points d'intersection déterminés.

Il s'agit par exemple de définir une zone englobant tous les points d'intersection correspondant à une même tâche visuelle.

Les zones d'usure peuvent être limitées par la géométrie de chaque monture. Elles peuvent dépendre des qualités de la lentille que l'on souhaite exploiter à travers elles.

Par exemple, pour une lentille ophtalmique progressive, sans limitation de la part de la monture, on pourrait utiliser une zone d'usure pour les visions de loin et de près faisant 60 degrés d'angle d'amplitude verticale et 60 degrés d'angle d'amplitude horizontale.

Le terminal informatique peut également être programmé pour déduire d'autres paramètres de conception optique de la lentille ophtalmique à partir des informations sur les directions de regard. Il peut déterminer la distance verticale entre la direction de regard moyenne dans une activité en vision de loin et la direction de regard moyenne dans une activité en vision de près, de manière à concevoir une lentille ophtalmique à addition progressive dans laquelle la distance entre la croix de montage et le centre de la zone de vision de près est identique à cette distance verticale déterminée.

La source lumineuse infra-rouge peut être de petite taille, par exemple mesurer environ 0,2 millimètre sur 0,2 millimètre et positionnée pour former une image dans le champ visuel central, que ce soit par éclairage direct ou via un guide d'onde, elle peut être non ou très faiblement visible, et permettre ainsi de ne pas altérer le confort de vision du porteur, ni d'introduire des objets parasites dans son champ de vision.

On peut envisager que l'anneau de support ne soit pas maintenu par une barre horizontale mais qu'il soit directement positionné sur la lentille ophtalmique ou la monture par un système de maintien par ventouse ou encore par des aimants positionnés de part et d'autres de la lentille ophtalmique ou de la monture, par des éléments adhésifs faibles repositionnables. On peut également envisager un maintien par capillarité (goutte d'eau, magic patches) ou un collage moléculaire sec via micro/nano structuration de surface, par exemple avec des nanofils de carbone.

## Revendications

1. Dispositif pour la détermination de la position d'un point remarquable d'un oeil (O1) d'un porteur équipé d'une monture de vision (400), comprenant
- au moins un appareil de capture d'image (120 ; 220 ; 320), et
- des moyens de positionnement pour positionner le dispositif de capture d'image par rapport à ladite monture de vision (400), de telle manière que, lorsque ladite monture de vision (400) est disposée en position utile sur la tête du porteur, l'appareil de capture d'image (120 ; 220 ; 320) est adapté à capturer une image de cet oeil (O1) du porteur, et
- des moyens de détermination de la position d'un point remarquable de l'oeil (O1) du porteur à partir de ladite au moins une image de l'oeil (O1) du porteur capturée par l'appareil de capture d'image (120 ; 220 ; 320),
**caractérisé en ce que** :
ladite monture de vision (400) comportant au moins deux cercles (411, 412) et/ou deux lentilles ophtalmiques (420), lesdits moyens de positionnement comprennent un support de mesure (150 ; 250 ; 350) muni d'une barre horizontale (151 ; 251 ; 351) et de deux bras latéraux (152 ; 252 ; 352), ainsi que deux clips (131, 132 ; 231, 232 ; 331, 332) appartenant à ladite barre horizontale et adaptés à coopérer avec une partie supérieure des cercles ou des lentilles ophtalmiques de la monture de vision (400), et deux clips (133, 134 ; 233, 234 ; 433, 434) disposés chacun à l'extrémité de l'un des bras latéraux (152 ; 252 ; 352) et adaptés à coopérer avec une partie inférieure des cercles (411, 412) ou des lentilles ophtalmiques (420) de la monture de vision (400).

2. Dispositif selon la revendication 1, dans lequel il est prévu en outre au moins une source lumineuse (110 ; 210 ; 310) agencée de manière à éclairer au moins un oeil (O1) du porteur, l'image capturée par l'appareil de capture d'image (120 ; 220 ; 320) comprenant alors une image d'au moins cet oeil (O1) du porteur éclairé par ladite source lumineuse (110 ; 210 ; 310).

3. Dispositif selon la revendication 2, dans lequel lesdits moyens de positionnement sont adaptés à positionner ledit dispositif par rapport à ladite monture de vision (400) de telle manière que, lorsque ladite monture de vision (400) est disposée en position utile sur la tête du porteur, la source lumineuse (110 ; 210 ; 310) est adaptée à éclairer l'oeil (O1) du porteur.

4. Dispositif selon l'une des revendications 2 et 3, dans lequel, la monture de vision (400) comprenant au moins une branche (414, 415) pour son installation sur la tête du porteur, les moyens de positionnement sont adaptés à coopérer avec cette monture de vision (400), de telle sorte que chaque source lumineuse (110 ; 210 ; 310) et chaque appareil de capture d'image (120 ; 220 ; 320) est disposé du côté de la monture de vision (400) où s'étend ladite branche (414, 415).

5. Dispositif selon l'une des revendications 2 à 4, dans lequelchaque appareil de capture d'image (120 ; 220 ; 320) est agencé de telle sorte qu'il est disposé en vis-à-vis du même cercle (411, 412) ou de la même lentille ophtalmique de la monture de vision (400) lorsque les moyens de positionnement coopèrent avec ladite monture de vision (400).

6. Dispositif selon l'une des revendications 2 à 4, dans lequel, il est prévu au moins deux sources lumineuses et deux appareils de capture d'image (120 ; 220 ; 320), agencés de telle sorte qu'au moins l'une des deux sources lumineuses et l'un des deux appareils de capture d'image (120 ; 220 ; 320) est disposé en vis-à-vis de chaque cercle (411, 412) ou de chaque lentille ophtalmique de la monture de vision (400) lorsque les moyens de positionnement coopèrent avec cette monture de vision (400).

7. Dispositif selon l'une des revendications 1 à 6, dans lequel chaque source lumineuse (110 ; 210 ; 310) et chaque appareil de capture d'image (120 ; 220 ; 320) est porté par l'un des bras latéraux (152 ; 252 ; 352) du support de mesure (150 ; 250 ; 350).

8. Dispositif selon l'une des revendications 1 à 7, dans lequel ledit support de mesure (250 ; 350) comprend en outre un anneau de support (253, 254 ; 354) qui est monté sur ladite barre horizontale (251, 351) et supporte ladite au moins une source lumineuse (210 ; 310) et ledit au moins un appareil de capture d'image (220 ; 320).

9. Dispositif selon la revendication 8, dans lequel ledit anneau de support (253, 254; 354) est monté sur ladite barre horizontale (251, 351) mobile en translation selon un axe parallèle à la droite reliant les deux clips (231, 232 ; 331, 332) de la barre horizontale (251, 351) et/ou mobile en translation selon un axe perpendiculaire à cette droite.

10. Dispositif selon l'une des revendications 8 à 9, dans lequel ledit support de mesure comporte en outre au moins un guide d'onde acheminant la lumière émise par ladite source lumineuse.

11. Dispositif pour le suivi de la direction de regard d'un porteur équipé d'une monture de vision (400), comprenant un dispositif pour la détermination de la position d'un point remarquable d'un oeil (O1) du porteur selon l'une des revendications 1 à 10, et des moyens de détermination de la direction de regard du porteur en fonction de la position de ce point remarquable de l'oeil (O1).

12. Procédé de détermination de la position d'un point remarquable d'au moins un oeil (O1) d'un porteur équipé d'une monture de vision (400), au moyen d'un dispositif pour la détermination de la position d'un point remarquable de l'oeil (O1) d'un porteur selon l'une des revendications 1 à 11, comportant au moins un appareil de capture d'image (120 ; 220 ; 320) agencé pour venir en vis-à-vis de cet oeil (O1) du porteur, comprenant les étapes suivantes :
a) positionner le dispositif pour la détermination de la position d'un point remarquable de l'oeil (O1) du porteur par rapport à la monture de vision (400) du porteur,
c) capturer au moins une image de l'oeil (O1) du porteur avec l'appareil de capture d'image (120 ; 220 ; 320) dudit dispositif de suivi,
d) déterminer la position du point remarquable de l'oeil (O1) à partir de l'image capturée.

13. Procédé de détermination de la position d'un point remarquable d'au moins un oeil (O1) d'un porteur selon la revendication 12, selon lequel, à l'étape a), on positionne le dispositif pour la détermination de la position d'un point remarquable de l'oeil (O1) du porteur par rapport à la monture de vision (400) du porteur de telle sorte que ledit au moins un appareil de capture d'image (120 ; 220 ; 320) soit adapté à capturer une image de l'oeil (O1) du porteur.

14. Procédé selon l'une des revendications 12 et 13, selon lequel, le dispositif pour la détermination de la position d'un point remarquable de l'oeil (O1) comportant au moins deux appareils de capture d'image (120 ; 220 ; 320) agencés pour venir en vis-à-vis de l'oeil (O1) du porteur, à l'étape d), on détermine la position du point remarquable de l'oeil (O1) par un calcul de triangulation à partir de deux images capturées simultanément par les deux dispositifs de capture d'image.

15. Procédé de détermination de la direction de regard d'au moins un oeil (O1) d'un porteur équipé d'une monture de vision (400), au moyen d'un dispositif pour le suivi de la direction de regard selon la revendication 11, comportant au moins un appareil de capture d'image (120 ; 220 ; 320) agencés pour venir en vis-à-vis de cet oeil (O1) du porteur, comprenant les étapes suivantes :
e) déterminer la position d'un point remarquable d'au moins un oeil (O1) du porteur selon le procédé de détermination de l'une des revendications 12 à 14,
f) déterminer la direction de regard du porteur en fonction de la position de ce point remarquable de l'oeil (O1).

## Patentansprüche

1. Vorrichtung zur Bestimmung der Position eines markanten Punkts eines Auges (01) eines Trägers, der mit einem Sehgestell (400) ausgestattet ist, umfassend
- wenigstens eine Bildaufnahmeeinrichtung (120; 220; 320) und
- Positionierungsmittel, um die Bildaufnahmevorrichtung bezogen auf das Sehgestell (400) so zu positionieren, dass, wenn das Sehgestell (400) in Gebrauchsposition auf dem Kopf des Trägers angeordnet ist, die Bildaufnahmeeinrichtung (120; 220; 320) geeignet ist, ein Bild dieses Auges (01) des Trägers aufzunehmen, und
- Mittel zur Bestimmung der Position eines markanten Punkts des Auges (01) des Trägers anhand des wenigstens einen Bilds des Auges (01) des Trägers, das von der Bildaufnahmeeinrichtung (120; 220; 320) aufgenommen wird
**dadurch gekennzeichnet, dass**:
das Sehgestell (400) aufweisend wenigstens zwei ophthalmische Fassungen (411, 412) und/oder Linsen (420), die Positionierungsmittel einen Messträger (150; 250; 350) mit einem horizontalen Balken (151; 251; 351) und zwei Seitenarmen (152; 252; 352) sowie zwei Clips (131, 132; 231; 232; 331; 332), die zum horizontalen Balken gehören und geeignet sind, mit einem oberen Abschnitt der ophthalmischen Fassungen oder Linsen des Sehgestells (400) zusammenzuwirken, und zwei Clips (133, 134; 233, 234; 433, 434), die jeweils am Ende eines der Seitenarme (152; 252; 352) angeordnet sind und geeignet sind, mit einem unteren Abschnitt der ophthalmischen Fassungen (411, 412) oder Linsen (420) des Sehgestells (400) zusammenzuwirken, umfassen.

2. Vorrichtung nach Anspruch 1, wobei ferner wenigstens eine Lichtquelle (110; 210; 310) vorgesehen ist, die so angeordnet ist, dass sie wenigstens ein Auge (01) des Trägers beleuchtet, wobei das Bild, das von der Bildaufnahmeeinrichtung (120; 220; 320) aufgenommen wird, dann ein Bild wenigstens dieses Auges (01) des Trägers umfasst, das von der Lichtquelle (110; 210; 310) beleuchtet wird.

3. Vorrichtung nach Anspruch 2, wobei die Positionierungsmittel geeignet sind, die Vorrichtung bezogen auf das Sehgestell (400) so zu positionieren, dass, wenn das Sehgestell (400) in Gebrauchsposition auf dem Kopf des Trägers angeordnet ist, die Lichtquelle (110; 210; 310) geeignet ist, das Auge (01) des Trägers zu beleuchten.

4. Vorrichtung nach einem der Ansprüche 2 und 3, wobei, das Sehgestell (400) umfassend wenigstens einen Bügel (414, 415) für seinen Sitz auf dem Kopf des Trägers, die Positionierungsmittel geeignet sind, so mit diesem Sehgestell (400) zusammenzuwirken, dass jede Lichtquelle (110; 210; 310) und jede Bildaufnahmeeinrichtung (120; 220; 320) auf der Seite des Sehgestells (400) angeordnet ist, wo sich der Bügel (414, 415) erstreckt.

5. Vorrichtung nach einem der Ansprüche 2 bis 4, wobei jede Bildaufnahmeeinrichtung (120; 220; 320) so angeordnet ist, dass sie gegenüber derselben ophthalmischen Fassung (411, 412) oder Linse des Sehgestells (400) angeordnet ist, wenn die Positionierungsmittel mit dem Sehgestell (400) zusammenwirken.

6. Vorrichtung nach einem der Ansprüche 2 bis 4, wobei wenigstens zwei Lichtquellen und zwei Bildaufnahmeeinrichtungen (120; 220; 320) vorgesehen sind, die so angeordnet sind, dass wenigstens eine der zwei Lichtquellen und eine der zwei Bildaufnahmeeinrichtungen (120; 220; 320) gegenüber jeder ophthalmischen Fassung (411, 412) oder Linse des Sehgestells (400) angeordnet ist, wenn die Positionierungsmittel mit diesem Sehgestell (400) zusammenwirken.

7. Vorrichtung nach einem der Ansprüche 1 bis 6, wobei jede Lichtquelle (110; 210; 310) und jede Bildaufnahmeeinrichtung (120; 220; 320) von einem der Seitenarme (152; 252; 352) des Messträgers (150; 250; 350) getragen wird.

8. Vorrichtung nach einem der Ansprüche 1 bis 7, wobei der Messträger (250; 350) ferner einen Tragring (253, 254; 354) umfasst, der auf dem horizontalen Balken (251, 351) angebracht ist und die wenigstens eine Lichtquelle (210; 310) und die wenigstens eine Bildaufnahmeeinrichtung (220; 320) trägt.

9. Vorrichtung nach Anspruch 8, wobei der Tragring (253, 254; 354) auf dem horizontalen Balken (251, 351) gemäß einer Achse parallel zu der Geraden, die die zwei Clips (231, 232; 331, 332) des horizontalen Balkens (251, 351) verbindet, und/oder gemäß einer Achse senkrecht zu dieser Geraden translatorisch beweglich angebracht ist.

10. Vorrichtung nach einem der Ansprüche 8 bis 9, wobei der Messträger ferner wenigstens einen Wellenleiter aufweist, der das Licht weiterleitet, das von der Lichtquelle abgegeben wird.

11. Vorrichtung zum Verfolgen der Blickrichtung eines Trägers, der mit einem Sehgestell (400) ausgestattet ist, umfassend eine Vorrichtung zur Bestimmung der Position eines markanten Punkts eines Auges (01) des Trägers nach einem der Ansprüche 1 bis 10 und Mittel zur Bestimmung der Blickrichtung des Trägers in Abhängigkeit von der Position dieses markanten Punkts des Auges (01).

12. Verfahren zur Bestimmung der Position eines markanten Punkts wenigstens eines Auges (01) eines Trägers, der mit einem Sehgestell (400) ausgestattet ist, mittels einer Vorrichtung zur Bestimmung der Position eines markanten Punkts des Auges (01) eines Trägers nach einem der Ansprüche 1 bis 11, die wenigstens eine Bildaufnahmeeinrichtung (120; 220; 320) aufweist, die angeordnet ist, sich diesem Auge (01) des Trägers gegenüber zu befinden, umfassend die folgenden Schritte:
a) Positionieren der Vorrichtung zur Bestimmung der Position eines markanten Punkts des Auges (01) des Trägers bezogen auf das Sehgestell (400) des Trägers,
c) Aufnehmen wenigstens eines Bilds des Auges (01) des Trägers, mit der Bildaufnahmeeinrichtung (120; 220; 320) der Verfolgungsvorrichtung,
d) Bestimmen der Position des markanten Punkts des Auges (01) anhand des aufgenommenen Bilds.

13. Verfahren zur Bestimmung der Position eines markanten Punkts wenigstens eines Auges (01) eines Trägers nach Anspruch 12, wobei bei Schritt a) die Vorrichtung zur Bestimmung der Position eines markanten Punkts des Auges (01) des Trägers bezogen auf das Sehgestell (400) des Trägers so positioniert wird, dass die wenigstens eine Bildaufnahmeeinrichtung (120; 220; 320) geeignet ist, ein Bild des Auges (01) des Trägers aufzunehmen.

14. Verfahren nach einem der Ansprüche 12 und 13, wobei, die Vorrichtung zur Bestimmung der Position eines markanten Punkts des Auges (01) aufweisend wenigstens zwei Bildaufnahmeeinrichtungen (120; 220; 320), die angeordnet sind, sich dem Auge (01) des Trägers gegenüber zu befinden, bei Schritt d) die Position des markanten Punkts des Auges (01) durch eine Triangulationsberechnung anhand zweier Bilder bestimmt wird, die simultan von den zwei Bildaufnahmeeinrichtungen aufgenommen werden.

15. Verfahren zur Bestimmung der Blickrichtung wenigstens eines Auges (01) eines Trägers, der mit einem Sehgestell (400) ausgestattet ist, mittels einer Vorrichtung zum Verfolgen der Blickrichtung nach Anspruch 11, die wenigstens eine Bildaufnahmeeinrichtung (120; 220; 320) aufweist, die angeordnet ist, sich diesem Auge (01) des Trägers gegenüber zu befinden, umfassend die folgenden Schritte:
e) Bestimmen der Position eines markanten Punkts wenigstens eines Auges (01) des Trägers gemäß dem Bestimmungsverfahren eines der Ansprüche 12 bis 14,
f) Bestimmen der Blickrichtung des Trägers in Abhängigkeit von der Position dieses markanten Punkts des Auges (01).

## Claims

1. Device for determining the position of a noteworthy point of an eye (01) of a wearer equipped with a vision frame (400), comprising
- at least one image-capturing apparatus (120; 220; 320), and
- positioning means for positioning the image-capturing device with respect to said vision frame (400), in such a way that, when said vision frame (400) is placed in useful position on the head of the wearer, the image-capturing apparatus (120; 220; 320) is suitable for capturing an image of this eye (01) of the wearer, and
- means for determining the position of a noteworthy point of the eye (01) of the wearer from said at least one image of the eye (01) of the wearer which was captured by the image-capturing apparatus (120; 220; 320),
**characterized in that**:
said vision frame (400) including at least two rims (411, 412) and/or two ophthalmic lenses (420), said positioning means comprise a measuring support (150; 250; 350) that is equipped with a horizontal bar (151; 251; 351) and two lateral arms (152; 252; 352), and two clips (131, 132; 231, 232; 331, 332) belonging to said horizontal bar and suitable for interacting with an upper portion of the rims or ophthalmic lenses of the vision frame (400), and two clips (133, 134; 233, 234; 433, 434) that are each placed at the end of one of the lateral arms (152; 252; 352) and suitable for interacting with a lower portion of the rims (411, 412) or of the ophthalmic lenses (420) of the vision frame (400).

2. Device according to Claim 1, wherein provision is furthermore made for at least one light source (110; 210; 310) arranged so as to illuminate at least one eye (01) of the wearer, the image captured by the image-capturing apparatus (120; 220; 320) then comprising an image of at least this eye (01) of the wearer illuminated by said light source (110; 210; 310).

3. Device according to Claim 2, wherein said positioning means are suitable for positioning said device with respect to said vision frame (400) in such a way that, when said vision frame (400) is placed in useful position on the head of the wearer, the light source (110; 210; 310) is suitable for illuminating the eye (01) of the wearer.

4. Device according to one of Claims 2 and 3, wherein, the vision frame (400) comprising at least one frame temple (414, 415) for its installation on the head of the wearer, the positioning means are suitable for interacting with this vision frame (400), such that each light source (110; 210; 310) and each image-capturing apparatus (120; 220; 320) is placed on the side of the vision frame (400) on which said frame temple (414, 415) extends.

5. Device according to one of Claims 2 to 4, wherein each image-capturing apparatus (120; 220; 320) is arranged such that it is placed facing the same rim (411, 412) or the same ophthalmic lens of the vision frame (400) when the positioning means are interacting with said vision frame (400).

6. Device according to one of Claims 2 to 4, wherein provision is made for at least two light sources and two image-capturing apparatuses (120; 220; 320), which are arranged such that at least one of the two light sources and one of the two image-capturing apparatuses (120; 220; 320) is placed facing each rim (411, 412) or each ophthalmic lens of the vision frame (400) when the positioning means are interacting with this vision frame (400) .

7. Device according to one of Claims 1 to 6, wherein each light source (110; 210; 310) and each image-capturing apparatus (120; 220; 320) is borne by one of the lateral arms (152; 252; 352) of the measuring support (150; 250; 350).

8. Device according to one of Claims 1 to 7, wherein said measuring support (250; 350) furthermore comprises a supporting ring (253, 254; 354) that is mounted on said horizontal bar (251, 351) and supports said at least one light source (210; 310) and said at least one image-capturing apparatus (220; 320).

9. Device according to Claim 8, wherein said supporting ring (253, 254; 354) is mounted on said horizontal bar (251, 351) so as to be translationally movable along an axis parallel to the straight line joining the two clips (231, 232; 331, 332) of the horizontal bar (251, 351) and/or translationally movable along an axis perpendicular to this straight line.

10. Device according to one of Claims 8 to 9, wherein said measuring support furthermore includes at least one waveguide guiding the light emitted by said light source.

11. Device for tracking the gaze direction of a wearer equipped with a vision frame (400), comprising a device for determining the position of a noteworthy point of an eye (01) of the wearer according to one of Claims 1 to 10, and means for determining the gaze direction of the wearer depending on the position of this noteworthy point of the eye (01).

12. Method for determining the position of a noteworthy point of at least one eye (01) of a wearer equipped with a vision frame (400), by means of a device for determining the position of a noteworthy point of the eye (01) of a wearer according to one of Claims 1 to 11, including at least one image-capturing apparatus (120; 220; 320) that is arranged to be facing this eye (01) of the wearer, comprising the following steps:
a) positioning the device for determining the position of a noteworthy point of the eye (01) of the wearer with respect to the vision frame (400) of the wearer,
c) capturing at least one image of the eye (01) of the wearer with the image-capturing apparatus (120; 220; 320) of said tracking device,
d) determining the position of the noteworthy point of the eye (01) from the captured image.

13. Method for determining the position of a noteworthy point of at least one eye (01) of a wearer according to Claim 12, wherein, in step a), the device for determining the position of a noteworthy point of the eye (01) of the wearer is positioned with respect to the vision frame (400) of the wearer such that said at least one image-capturing apparatus (120; 220; 320) is suitable for capturing an image of the eye (01) of the wearer.

14. Method according to one of Claims 12 and 13, wherein, the device for determining the position of a noteworthy point of the eye (01) including at least two image-capturing apparatuses (120; 220; 320) that are arranged so as to be facing the eye (01) of the wearer, in step d), the position of the noteworthy point of the eye (01) is determined via a triangulation calculation from two images that are captured simultaneously by the two image-capturing devices.

15. Method for determining the gaze direction of at least one eye (01) of a wearer equipped with a vision frame (400), by means of a device for tracking the gaze direction according to Claim 11, including at least one image-capturing apparatus (120; 220; 320) that is arranged to be facing this eye (01) of the wearer, comprising the following steps:
e) determining the position of a noteworthy point of at least one eye (01) of the wearer using the determining method of one of Claims 12 to 14,
f) determining the gaze direction of the wearer depending on the position of this noteworthy point of the eye (01).
